# EUROPEAN PATENT APPLICATION

(11) **EP 1 466 982 A1**
(43) Date of publication of application: **13.10.2004**
(21) Application number: 02792009.9
(22) Date of filing: 26.12.2002
(51) Int. Cl.: C12N 15/57, C12N 9/62, C12N 1/15, C12N 1/19, C12N 1/21, C12N 5/00, C12P 21/02, C07K 16/14, G01N 33/53

(54) **PYROGLUTAMYL PEPTIDASE AND ITS GENE**

(30) Priority: 27.12.2001 JP 2001403261
(71) Applicant: National Institute of Advanced Industrial Science and Technology, Tokyo 100-8921 (JP); National Institute of Technology and Evaluation, Tokyo (JP); National Research Institute of Brewing, Higashihiroshima-shi, Hiroshima 739-0046 (JP); KYOWA HAKKO KOGYO CO., LTD., Chiyoda-ku, Tokyo 100-8185 (JP)
(72) Inventor: MACHIDA, Masayuki, Tsukuba-shi (JP); ABE, Keietsu, Shiogama-shi, Miyagi 985-0043 (JP); GOMI, Katsuya, Sendai-shi, Miyagi 989-3201 (JP); ASAI, Kiyoshi, Tokyo (JP); SANO, Motoaki, Tsukuba-shi, Tokyo (JP); KIN, Taishin, Tokyo (JP); NAGASAKI, Hideki, Tokyo (JP); HOSOYAMA, Akira, Nat. Inst. Of Tech. & Evaluation, Tokyo 151-0066 (JP); AKITA, Osamu, National Institute of Brewing, Higashihiroshima-shi, Hiroshima 739-0046 (JP); OGASAWARA, Naotake, Ikoma-shi, Nara 630-0243 (JP); KUHARA, Satoru, Fukuoka-shi, Fukuoka 811-1355 (JP); TOKUNAGA, Chikara, Kyowa Hakko Kogyo Co.,Ltd., Inashiki-gun, Ibaraki 300-0398 (JP); TODA, Itaru, Kyowa Hakko Kogyo Co.,Ltd., Inashiki-gun, Ibaraki 300-0398 (JP); SAITOH, Chiaki, Kyowa Hakko Kogyo Co.,Ltd., Inashiki-gun, Ibaraki 300-0398 (JP); SENOH, Akihiro, Machida-shi, Tokyo 194-0021 (JP)
(74) Representative: VOSSIUS & PARTNER
(86) International application number: PCT/JP2002/013627
(87) International publication number: WO 2003/056018

(57) **Abstract**

The present invention provides DNA encoding novel pyroglutamyl peptidase derived from *Aspergillus oryzae,* pyroglutamyl peptidase which is produced by using the DNA, and a method for producing a protein lysate with a good flavor at a high hydrolysis rate.

## Description

### TECHNICAL FIELD

The present invention relates to a pyroglutamyl peptidase used in production of protein hydrolysates and DNA encoding the pyroglutamyl peptidase.

### BACKGROUND ART

Among filamentous fungi, in particular, Koji molds including *Aspergillus oryzae* has been used for a long time in the field of fermented food production in our country, which produces sake, bean paste, soy sauce, Japanese sweet rice wine for cooking, etc., and thus, this fungus has been directly eaten. Koji molds are safe gene sources that have been listed up as GRAS (Generally Recognized as Safe) by the FDA (Food and Drug Administration) of USA. Thus, filamentous fungi, and especially, Koji molds are considered to be a treasure trove of genes with extremely high utility value in terms of safety.

Protein hydrolysates can be produced by hydrolysis of a raw material comprising a protein with acid. From the viewpoint of the use of protein hydrolysates as natural seasonings, a production process of protein hydrolysates by enzymolysis is being studied, in addition to the production method thereof by acidolysis. As protein hydrolysates produced by enzymolysis, enzymatic hydrolysate of albumen (Japanese Published Unexamined Patent Application No. 68773/73), enzymatic hydrolysate of defatted soybeans (Japanese Published Unexamined Patent Application No. 70852/76), enzymatic hydrolysate of cheese whey as a raw material (Japanese Published Unexamined Patent Application No. 151155/87), and enzymatic hydrolysate of corn gluten meal (Japanese Published Examined Patent Application No. 295437/90) have been reported.

However, there may be a case where a generated peptide has a bitter taste depending on properties of a protein and enzymes used in proteolysis and therefore it is not functionally preferred. Thus, a hydrolysate having a high degradation ratio in hydrolysis and excellent functional properties has been desired. In order to improve such a degradation rate, techniques applied to various enzymes such as glutaminase derived from Koji molds (WO99/60104, Japanese Published Unexamined Patent Application No. 166547/2000, and Japanese National Publication of International Patent Application No. 511746/2002) are being considered. As shown in the fermentation of soy sauce and bean paste, in the case of using the conventional Aspergillus culture products, although the hydrolysis of proteins requires enormous manpower and time, it results in a low releasing rate of amino acid. In particular, it results in a low releasing rate of glutamic acid, which is contained in the largest amount in soy protein and is important for flavoring.

In the mean time, there are a large number of proteins and peptides whose N terminus is protected by an L-pyroglutamic acid residue. Moreover, when a protein or peptide is hydrolyzed, glutamine or glutamic acid at a newly generated amino terminus is nonenzymatically cyclized to form a pyroglutamic acid residue in many cases, and such a residue is detected also in food. Since these proteins or peptides whose N terminus is protected by an L-pyroglutamic acid residue are not directly subjected to hydrolysis by amino peptidase, they require an operation to eliminate the L-pyroglutamic acid residue. Pyroglutamyl peptidase is an enzyme for specifically releasing an L-pyroglutamic acid residue located at an amino terminus of these proteins or peptides. It has been known that this enzyme widely exists in a wide range of areas such as the brain, lung, serum or pituitary gland of various animals, plants, or microorganisms. It has been reported that a protein hydrolysate obtained by reacting a protein with protein hydrolase is further reacted with pyroglutamyl peptidase, so as to produce a protein hydrolysate with good flavoring properties (Japanese Published Unexamined Patent Application No.252075/96).

An enzyme derived from *Bacillus amyloliquefaciens* has been known as pyroglutamyl peptidase derived from microorganisms [J. Biochem., 84, 467 (1978)]. With regard to this enzyme, its gene has been isolated, and a production method thereof has been reported (Japanese Published Unexamined Patent Application No. 137572/93). Moreover, with regard to an enzyme with high heat resistance derived from *Pyrococcus furiosus* also, its gene has been isolated (Japanese Published Unexamined Patent Application No. 298881/95). Furthermore, an enzyme derived from *Bacillus subtilis* has been known (Japanese Published Unexamined Patent Application No.252075/96). However, pyroglutamyl peptidase derived from filamentous fungi and a gene thereof have not been isolated yet. Still further, in case of using pyroglutamyl peptidase together with a culture product of Koji mold as a protein hydrolase source in the production of protein hydrolysates, pyroglutamyl peptidase derived from heterologous organisms other than Koji molds, such as *Bacillus subtilis,* has been used. It is considered that it results in high cost and deteriorated flavor.

### DISCLOSURE OF THE INVENTION

The present invention provides: a novel pyroglutamyl peptidase derived from filamentous fungi, which can be used in the production of a protein hydrolysate having a higher releasing rate of amino acid, and especially, a higher releasing rate of glutamic acid, than those of the conventional protein hydrolysates that are obtained by hydrolysis with enzymes; and a pyroglutamyl peptidase gene that can be used in the production of the pyroglutamyl peptidase.

That is to say, the present invention relates to polypeptides of the embodiments described below, and DNAs encoding the polypeptides. Among the polypeptides and DNAs of the present invention, those derived from *Aspergillus oryzae* which is a microorganism listed up as GRAS by the FDA of USA, have extremely high utility value in terms of safety and economical efficiency.

The present invention provides the following (1) to (23):
(1) A polypeptide selected from the group consisting of the following (a) to (c):
   (a) a polypeptide comprising an amino acid sequence shown in SEQ ID NO: 2,
   (b) a polypeptide comprising an amino acid sequence substantially identical to the amino acid sequence shown in SEQ ID NO: 2, and having a pyroglutamyl peptidase activity, and
   (c) a polypeptide comprising an amino acid sequence wherein one or more amino acid residues are deleted, substituted or added in the amino acid sequence shown in SEQ ID NO: 2, and having a pyroglutamyl peptidase activity.

The amino acid sequence shown in SEQ ID NO: 2 is the amino acid sequence of pyroglutamyl peptidase of *Aspergillus oryzae.*

The term "an amino acid sequence substantially identical to the amino acid sequence shown in SEQ ID NO: 2" is used herein to mean an amino acid sequence showing an average homology of approximately 30% or more, preferably approximately 50% or more, more preferably approximately 80% or more, and particularly preferably approximately 90% or more with the amino acid sequence shown in SEQ ID NO: 2, when the two amino acid sequences are aligned to compare the entirety of the sequences.

The term "an amino acid sequence wherein one or more amino acid residues are deleted, substituted or added in the amino acid sequence shown in SEQ ID NO: 2" is used herein to mean an amino acid sequence wherein preferably approximately 1 to 20, more preferably approximately 1 to 10, and further more preferably several amino acid residues are deleted, substituted or added, or amino acid sequence obtained by combining these amino acid sequences.

The pyroglutamyl peptidase activity of a polypeptide can be determined as follows. A 50 mmol/l phosphate buffer solution (pH 7.5) containing 5 mmol/l pyroglutamic acid-paranitroanilide is prepared as a substrate solution. A sample solution containing polypeptides is prepared. 20 µl of the sample solution is added to 100 µl of the substrate solution, and the mixture is reacted at 37°C for 10 minutes. Thereafter, an absorbance at 405 nm is determined using a spectrophotometer. The amount by which paranitroaniline has released per minute of reaction time is calculated from the molar absorption coefficient of paranitroaniline (10500). An activity to release 1 micromole of paranitroaniline at 37°C for 1 minute is defined as 1 unit.

The above polypeptide comprising an amino acid sequence substantially identical to the amino acid sequence shown in SEQ ID NO: 2, or polypeptide comprising an amino acid sequence wherein one or more amino acid residues are deleted, substituted or added in the amino acid sequence shown in SEQ ID NO: 2, can be easily produced by appropriately combining methods known to a person skilled in the art, such as a site-directed mutagenesis, gene homologous recombination, primer extension or PCR method.

In order that the produced polypeptide has substantially identical functions, it seems to be effective to substitute homologous amino acids (e.g., polar/nonpolar amino acids, hydrophobic/hydrophilic amino acids, positively charged/negatively charged amino acids, aromatic amino acids, etc.) with each other, among amino acids constituting the polypeptide. Moreover, in order to maintain the substantially identical functions, amino acids in functional domains contained in each polypeptide of the present invention are desirably retained.

An example of the polypeptide comprising an amino acid sequence wherein one or more amino acid residues are deleted, substituted or added in the amino acid sequence shown in SEQ ID NO: 2 and having a pyroglutamyl peptidase activity includes a polypeptide comprising an amino acid sequence shown in SEQ ID NO: 10, in which 41 amino acid residues are added to the N terminus of the amino acid sequence shown in SEQ ID NO: 2.
(2) DNA comprising a nucleotide sequence encoding the polypeptide according to (1).
(3) DNA selected from the group consisting of the following (a) to (c):
   (a) DNA comprising a nucleotide sequence shown in SEQ ID NO: 1,
   (b) DNA comprising a nucleotide sequence shown in SEQ ID NO: 5, and
   (c) DNA which hybridizes with DNA comprising a nucleotide sequence complementary to the nucleotide sequence shown in SEQ ID NO: 1 or 5 under stringent conditions, and comprises a nucleotide sequence encoding a polypeptide having a pyroglutamyl peptidase activity.

DNAs according to (2) and (3) have a function as a region which encodes a polypeptide having a pyroglutamyl activity. An example of an amino acid sequence encoded by these DNAs is shown in SEQ ID NO: 2. The amino acid sequence shown in SEQ ID NO: 2 has been determined from various information (ORF, exon/intron region, expressed sequence tag (EST), etc.) to identify a gene region (location of gene) on the basis of the genomic nucleotide sequence of *Aspergillus oryzae* RIB 40 (FERM P-18273 (transferred to FERM BP-7935)). The nucleotide sequence shown in SEQ ID NO: 1 is the nucleotide sequence of genomic DNA which comprises a pyroglutamyl peptidase gene of the *Aspergillus oryzae* RIB 40 and a promoter region thereof. The nucleotide sequence shown in SEQ ID NO: 5 is a nucleotide sequence obtained by removing 5' and 3' non-translation regions and introns from the nucleotide sequence shown in SEQ ID NO: 1, and it matches the nucleotide sequence of a region which encodes the pyroglutamyl peptidase of pyroglutamyl peptidase cDNA of the *Aspergillus oryzae* RIB 40.

In the present specification, the term "under stringent conditions" is used to mean conditions in which hybrids are specifically formed only between nucleotide sequences having a high homology, such as an average homology of approximately 80% or more, preferably approximately 90% or more, and more preferably approximately 95% or more. More specifically, an example of such stringent conditions includes conditions of a temperature between 60°C and 68°C, a sodium concentration of 150 to 900 mmol/l, and preferably 600 to 900 mmol/l, and pH of 6 to 8.

Hybridization can be carried out according to methods known in this field, such as the method described in Current Protocols in Molecular Biology, John Wiley & Sons (1987), or methods which are similar thereto. In addition, when a commercially available library is used, hybridization can be carried out according to the method described in instructions attached thereto. Examples of DNA which hybridizes with DNA comprising a nucleotide sequence complementary to the nucleotide sequence shown in SEQ ID NO: 1 or 5 under stringent conditions, and having a nucleotide sequence encoding a polypeptide having a pyroglutamyl peptidase activity, include genomic DNA and cDNA which encode pyroglutamyl peptidase derived from filamentous fungi other than *Aspergillus oryzae*.
(4) DNA selected from the group consisting of the following (a) to (c):
   (a) DNA comprising a nucleotide sequence shown in SEQ ID NO: 3,
   (b) DNA which comprises a partial sequence of the nucleotide sequence shown in SEQ ID NO: 3 consisting of 100 or more nucleotides and functions as a promoter, and
   (c) DNA which hybridizes with DNA comprising a nucleotide sequence complementary to the nucleotide sequence shown in SEQ ID NO: 3 under stringent conditions and functions as a promoter.

These DNA sequences correspond to regions which encode the polypeptide of the present invention in the genomic DNA of filamentous fungi, such as a region located 5' upstream of a nucleotide sequence corresponding to 1001 to 1111 nucleotides of the nucleotide sequence shown in SEQ ID NO: 1.

In some of these sequences, EST has actually been confirmed in a coding region located in a 3' downstream region thereof (Expression conditions: any one of a culture in a complete liquid medium containing 2% glucose; a culture in a complete liquid medium containing 2% maltose; a culture in synthetic liquid medium containing no carbon sources; a culture at a high temperature (37 °C, but in other cultures, a temperature of 30°C is applied unless otherwise specified) in a complete liquid medium containing 2% glucose; a solid culture (using wheat bran); a culture in an synthetic alkaline medium (pH 10) containing 2% glucose; a culture which was carried out immediately after germination of spores which had been cultured at 28°C for 8 days in a potato dextrose agar medium; and a solid culture at 25°C for 3 hours which was carried out after culturing for 34 hours in a mixed medium of soybeans and wheat). As is clear from this fact, some of these sequences comprise a promoter region (a sequence interacting with various polymerase, a general transcription factor, or a transcription factor, such as a core promoter or basic promoter and upstream promoter elements).

Accordingly, a sequence corresponding to preferably 200 or more base pairs, more preferably 500 or more base pairs, further more preferably 800 or more base pairs, and particularly preferably 900 or more base pairs from the 3' side of each of the above sequences, is suitable as a partial sequence that can comprise the above promoter region. Otherwise, a partial sequence having base pairs corresponding to the above length in a suitable intermediate portion in each of the above sequences can also be used.

It can be confirmed whether or not DNA functions as a promoter, for example, by the following method. DNA to be tested is ligated upstream of DNA encoding a polypeptide as a reporter, so as to prepare new DNA. The obtained DNA is inserted into a suitable vector comprising a transformation marker gene such as an acetamidase gene of *Aspergillus oryzae* or nitrate reductase gene [J. Ferment. Bioeng., 74, 389 (1992), Mol. Gen. Genet., 218, 99-104 (1989)], so as to produce a recombinant vector. *Aspergillus oryzae* is transformed with the obtained recombinant vector by the method described in the publications [J. Ferment. Bioeng., 74, 389 (1992), Mol. Gen. Genet., 218, 99-104 (1989)]. The level of a polypeptide as a reporter is measured in the obtained transformant. When such a polypeptide is detected, it is confirmed that the DNA ligated upstream of the DNA encoding the polypeptide acting as a reporter functions as a promoter. Examples of a polypeptide as a reporter may include β-glucuronidase of *Escherichia coli,* a green fluorescent protein, and β-galactosidase of *Escherichia coli.* The β-glucuronidase, green fluorescent protein and β-galactosidase in the transformant or a culture supernatant thereof can be detected according to the method described in the publications [Appl. Environ. Microbiol. 61, 2482 (1995), Eur. J. Biochem. 266, 252 (1999), Mol. Microbiol. 8, 211 (1993)].

The DNA according to (4) is also useful, for example, as a region into which a foreign gene or the like is inserted and expressed.
(5) DNA selected from the group consisting of the following (a) to (c):
   (a) DNA comprising a nucleotide sequence complementary to a nucleotide sequence shown in SEQ ID NO: 4,
   (b) DNA which comprises a partial sequence of a nucleotide sequence complementary to the nucleotide sequence shown in SEQ ID NO: 4 consisting of 10 or more nucleotides, and
   (c) DNA which hybridizes with DNA comprising the nucleotide sequence shown in SEQ ID NO: 4 under stringent conditions.

This DNA is DNA of a 3' non-translation region comprising nucleotides 1902 to 2201 of the nucleotide sequence shown in SEQ ID NO: 1 in the genomic DNA of *Aspergillus oryzae* RIB 40. In particular, when the DNA is hybridized with mRNA transcribed from a pyroglutamyl peptidase gene comprising the nucleotide sequence shown in SEQ ID NO: 1 and the mRNA is detected, this DNA can be used as a probe for the detection. The region encoding pyroglutamyl peptidase in the above mRNA, which corresponds to the nucleotide sequence shown in SEQ ID NO: 5, often comprises sequences having a high homology with other mRNAs in association with the functions of polypeptides encoded thereby. In contrast, the nucleotide sequence of this region comprises a highly arbitrary sequence that is totally irrelevant to SEQ ID NO: 5. Accordingly, using the DNA according to (5) as a probe, it is possible to differentially detect and quantify mRNA of pyroglutamyl peptidase with extremely high properties from a population of RNAs extracted from cells. Moreover, it is also possible to produce primers for PCR having the sequence of this region and to carry out such differential detection and quantification by PCR.

A portion corresponding to preferably 300 base pairs, more preferably 200 base pairs, and particularly preferably approximately 100 base pairs from the 5' side of the above sequence is suitable for the DNA of the present invention used as a probe, or a region corresponding to a partial sequence thereof. In addition, the length of such a partial sequence can be appropriately selected by a person skilled in the art, depending on intended use or the like. In general, it is preferred that the length of the sequence is longer within the above ranges in terms of detectability and quantitative sensitivity.
(6) The DNA according to any one of (2) to (5), wherein DNA is genomic DNA.
(7) An oligonucleotide comprising a nucleotide sequence consisting of 15 or more continuous nucleotides of the nucleotide sequence of the DNA according to any one of (2) to (6) above or a nucleotide sequence complementary thereto.
(8) A recombinant DNA comprising the DNA according to (2) or (3).
(9) A transformant comprising the recombinant DNA according to (8).
(10) A process for producing the polypeptide according to (1), which comprises culturing a microorganism having an ability to produce the polypeptide in a medium, so as to produce and accumulate the polypeptide in a culture, and recovering the polypeptide from the culture.
(11) The process according to (10), wherein the microorganism is the transformant according to (9).
(12) The process according to (10), wherein the microorganism is filamentous fungus.
(13) The process according to (12), wherein the filamentous fungus belongs to one genus selected from a group consisting of *Aspergillus, Penicillium, Humicola, Trichoderma, Mucor,* and *Fusarium.*
(14) The process according to (13), wherein the filamentous fungus belonging to *Aspergillus* belongs to one species selected from a group consisting of *Aspergillus oryzae*, *Aspergillus sojae, Aspergillus niger, Aspergillus awamori, Aspergillus kawachii, Aspergillus parasiticus, Aspergillus flavus, Aspergillus nomius, Aspergillus fumigatus,* and *Aspergillus nidulans.*
(15) A process for producing a protein hydrolysate, which comprises adding the polypeptide according to (1) and a protein hydrolase to a raw material containing a protein, and degrading the protein.
(16) A process for producing a protein hydrolysate, which comprises adding a culture containing the polypeptide according to (1) which is obtained by culturing a microorganism having an ability to produce the polypeptide according to (1) in a medium, or a treated product thereof, and a protein hydrolase, to a raw material containing a protein, and degrading the protein.
(17) The process according to (16), wherein the microorganism is the transformant according to (9).
(18) The process according to (16), wherein the microorganism is filamentous fungus.
(19) The process according to (18), wherein the filamentous fungus belongs to one genus selected from a group consisting of *Aspergillus, Penicillium, Humicola, Trichoderma*, *Mucor,* and *Fusarium.*
(20) The process according to (19), wherein the filamentous fungus belonging to *Aspergillus* belongs to one species selected from a group consisting of *Aspergillus oryzae, Aspergillus sojae, Aspergillus niger, Aspergillus awamori, Aspergillus kawachii, Aspergillus parasiticus, Aspergillus flavus, Aspergillus nomius, Aspergillus fumigatus,* and *Aspergillus nidulans.*
(21) A protein hydrolysate which is produced by the process according to any one of (15) to (20).
(22) An antibody which specifically binds to the polypeptide according to (1).
(23) A method of detecting or quantifying the polypeptide according to (1), which comprises using the antibody according to (22).

Embodiments of the present invention will be described in detail below.

### (1) Production of DNA of the present invention

With regard to DNA of the present invention, for example, DNA comprising a nucleotide sequence shown in SEQ ID NO: 1 can be produced, for example, by the shut gun cloning method described in Examples, using the genome of *Aspergillus oryzae* as a starting material. In the production, each fragmented chromosomal DNA is ligated to a suitable cloning vector such as a plasmid vector or phage, depending on its length or the like. Thereafter, a suitable host cell such as *Escherichia coli* is transformed therewith by an appropriate method such as the electroporation, so that a clone library for cloning the above fragmented chromosomal DNA can be produced.

Moreover, according to known methods such as a chemical decomposition method (Maxam-Gilbert method) or dideoxy method, the nucleotide sequence of each fragmented chromosomal DNA obtained from the clone library can be determined.

Otherwise, the DNA of the present invention can also be produced by amplification by PCR using, as primers, oligo DNA comprising a nucleotide sequence consisting of 15 or more contiguous nucleotides of the nucleotide sequence shown in SEQ ID NO: 1 or 5 or a nucleotide sequence complementary thereto. For example, using oligo DNA comprising nucleotide sequences shown in SEQ ID NOS: 8 and 9 as a primer set, PCR is carried out using the cDNA of *Aspergillus oryzae* as a template, so that DNA comprising the nucleotide sequence shown in SEQ ID NO: 5 can be amplified and isolated.

Moreover, PCR is carried out using, as a primer set, oligo DNA comprising a nucleotide sequence consisting of 15 or more contiguous nucleotides of the nucleotide sequence of the DNA of the present invention and oligo DNA comprising a nucleotide sequence consisting of 15 or more contiguous nucleotides of a nucleotide sequence complementary to the nucleotide sequence of the DNA of the present invention, so that the DNA fragment of the present invention can be amplified, and can be detected or isolated. With regard to primers, a region to be amplified is selected, and DNA comprising a sequence of 15 to 50 nucleotides of the 5' terminus of the region at the 3' terminus and DNA comprising a sequence complementary to a sequence of 15 to 50 nucleotides of the 3' terminus of the region at the 3' terminus are preferably produced and used as primers. As a template, for example, chromosomal DNA or cDNA of a microorganism, and preferably of filamentous fungus, can be used. Examples of preferred filamentous fungi include microorganisms belonging to any one genus selected from *Aspergillus, Penicillium, Humicola, Trichoderma, Mucor* and *Fusarium.* Of these, filamentous fungi belonging to *Aspergillus* may be particularly preferred. Examples of filamentous fungi belonging to *Aspergillus* include *Aspergillus oryzae, Aspergillus fumigatus, Aspergillus flavus, Aspergillus sojae, Aspergillus parasiticus, Aspergillus nomius, Aspergillus niger, Aspergillus awamori*, *Aspergillus kawachii,* and *Aspergillus nidulans*. Of these, filamentous fungi belonging to the flavi section are preferred. Examples of filamentous fungi of *Aspergillus* belonging to the flavi section include *Aspergillus oryzae, Aspergillus sojae, Aspergillus parasiticus, Aspergillus flavus,* and *Aspergillus nomius.* Of these, for example, *Aspergillus oryzae* RIB 40 (ATCC No. 42149) was deposited with the National Institute of Advanced Industrial Science and Technology, an Independent Administrative Institution under the Ministry of Economy, Trade and Industry, at the AIST, Tsukuba Central 6, Higashi 1-1-1, Tsukuba, Ibaraki, Japan) under accession No. FERM P-18273 on March 28, 2001. It was then transferred to an international deposition on March 4, 2002, and received an accession No. FERM BP-7935.

DNA comprising a nucleotide sequence shown in SEQ ID NO: 3 or 4 is a partial fragment of DNA having the nucleotide sequence shown in SEQ ID NO: 1, and it can be amplified and isolated by PCR using the genomic DNA of *Aspergillus oryzae* as a template and a primer set that is based on the nucleotide sequences shown in SEQ ID NOS: 3 and 4.

PCR can be carried out by applying conditions and means known to a person skilled in the art. As an example of PCR reaction conditions, after heating at 94°C for 2 minutes, a reaction cycle consisting of 94°C for 10 seconds, 55°C for 20 seconds, and 72°C for 2 minutes is repeated 30 times, and finally the reaction product is heated at 72°C for 5 minutes. As another example of PCR reaction conditions, after heating at 94°C for 5 minutes, a reaction cycle consisting of 94°C for 2 minutes, 56°C for 30 seconds, and 72°C for 1 minute 30 seconds is repeated 30 times. A common thermal cycler such as 9600 manufactured by Perkin Elmer or Program Temp Control System PC-700 manufactured by Astec Inc. can be used as a thermal cycler. As heat-resistant DNA polymerase, a commercially available product such as Taq DNA polymerase (manufactured by Takara Shuzo Co., Ltd.) or ExTaq DNA polymerase (manufactured by Takara Shuzo Co., Ltd.) is used, and the composition of a reaction solution can be determined in accordance with an instruction manual attached to the product.

The length of each primer consisting of nucleotides used for the above primer set for DNA amplification is not particularly limited, but it can be appropriately selected by a person skilled in the art depending on intended use or the like. A primer has a length of generally 15 to 50 nucleotides, and preferably 20 to 30 nucleotides. As the number of primers, a minimum number can be determined taking into consideration the degree of relationship between a strain containing DNA to be amplified and Aspergillus, and a mixed degree thereof. The number of primers is at least 1 set (2 primers), and preferably 2 to 4 sets. Moreover, in order to design primers, the length and properties of a sequence to be amplified are considered. Oligo DNA can be produced by chemical synthesis known to a person skilled in the art, for example, by using a DNA Synthesizer manufactured by Applied Biosystems.

A partial fragment of the DNA of the present invention, or oligo DNA comprising the nucleotide sequence of the DNA of the present invention or a nucleotide sequence consisting of 15 or more contiguous nucleotides of a nucleotide sequence complementary to the nucleotide sequence of the DNA of the present invention, is labeled with radioisotope, digoxigenin, biotin or the like, and it is used as a probe. Hybridization is carried out using such a probe, so as to detect the DNA of the present invention or mRNA encoding the polypeptide of the present invention. The partial fragment of the DNA of the present invention can be produced by PCR as described above, and oligo DNA can be produced by chemical synthesis or using a DNA synthesizer as in the case of the oligo DNA used as primers. The length of a probe can be appropriately selected by a person skilled in the art, considering a detection target or the like. The probe has a length of generally 15 to 3000 nucleotides, and preferably 20 to 1000 nucleotides.

To carry out hybridization, for example, DNA or mRNA is transferred from electrophoresis gel or colonies onto a nitrocellulose or nylon membrane. Thereafter, it is reacted at 80°C for 2 hours in a vacuum or subjected to ultraviolet radiation treatment, so as to immobilize DNA onto the membrane. During this process, denaturation with an alkaline solution containing 0.5 mol/l NaOH and 1.5 mol/l NaCl, and neutralization with a solution containing 0.5 mol/l Tris-HCl (pH 7.5) and 3 mol/l NaCl, are carried out as necessary. The obtained membrane is prehybridized at 42°C for 2 hours in a hybridization solution containing 50% formamide, 4 x SSC, 50 mM HEPES-NaOH (pH 7.0), 10 x Denhardt's solution and 100 µg/ml salmon sperm DNA. Thereafter, hybridization is carried out at 42°C over day and night in the same above hybridization solution to which the above probe is added. The resultant membrane is washed for 2 minutes with 2 x SSC solution containing 0.1% SDS at room temperature 3 times, and then washed for 2 hours with a 0.1 x SSC solution containing 0.1% SDS at 50°C 3 times. The washed membrane is air-dried, and it is exposed to an X ray film at -70°C for a period of time between 2 hours and over day and night, and then developed for visualization. Alternatively, the DNA of the present invention or mRNA encoding the polypeptide of the present invention can also be detected by using a DNA chip in which oligo DNA or a DNA fragment is immobilized onto a substrate, and hybridizing the DNA chip with a labeled mRNA or DNA, followed by detecting the above DNA or mRNA as dots [Genome Res., 6, 639 (1996)].

### (2) Production of polypeptide of the present invention

The polypeptide of the present invention can be produced, for example, by the method described below, which involves introducing recombinant DNA comprising DNA encoding the polypeptide of the present invention into a host cell to produce a transformant, and culturing the transformant. The methods described in Molecular Cloning: A Laboratory Manual, 3^{rd} edition, Cold Spring Harbor Laboratory (2001), Current Protocols in Molecular Biology, John Wiley & Sons (1987) or the like, can be applied as examples of gene manipulations.

From the DNA of the present invention obtained in (1), DNA with an appropriate length comprising a region encoding the polypeptide of the present invention is produced. In addition, DNA is produced, as necessary, by substituting nucleotides of a nucleotide sequence corresponding to the region encoding the polypeptide of the present invention with other nucleotides, so as to obtain codons optimal for expression in a host cell.

A recombinant vector is produced by inserting the above DNA downstream of a promoter of a suitable expression vector.

The obtained recombinant vector is introduced into a host cell suitable for the expression vector.

Any host cell can be used as long as the desired nucleic acid sequence can be expressed therein, and examples of such a host cell include bacteria, yeasts, filamentous fungi, animal cells, insect cells, and plant cells.

As an expression vector, a vector, which can autonomously replicate in the above host cells or be integrated into a chromosome and comprises a promoter at a position which enables transcription of DNA encoding the polypeptide of the present invention, can be used.

When a prokatyote such as bacterium is used as a host cell, the recombinant vector comprising DNA encoding the polypeptide of the present invention is preferably a vector, which has such a structure that a promoter, a ribosome binding sequence, DNA encoding the polypeptide of the present invention and a transcription termination sequence are ligated, as well as being capable of autonomously replicating in the prokatyote. A gene for regulating the promoter may also be contained therein.

Examples of an expression vector include pGEMEX-1 (manufactured by Promega), pQE-30 (manufactured by Qiagen), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pTrS30 [produced from *Escherichia coli* JM109/pTrS30 (FERM BP-5407)], pGEX-5X-3 (manufactured by Amersham Bioscience), pET14 (manufactured by Novagen), pPROTet.E (manufactured by Clontech), and pRSET C (manufactured by Invitrogen).

Any promoter can be used as long as it functions in a host cell. Examples of such a promoter include promoters derived from *Escherichia coli,* phage or the like, such as a trp promoter (Pₜᵣₚ), lac promoter, P_{L} promoter, P_{R} promoter or T7 promoter. In addition, artificially designed and modified promoters such as a promoter (Pₜᵣₚ x 2) in which the two Pₜᵣₚs are combined in tandem, tac promoter, lacT7 promoter or letI promoter may also be used.

A plasmid in which a distance between a Shine-Dalgarno sequence as a ribosome binding sequence and an initiation codon is appropriately adjusted (e.g., 6 to 18 nucleotides) is preferably used.

The recombinant vector of the present invention does not necessarily require a transcription termination sequence for the expression of the DNA of the present invention. However, such a transcription termination sequence is preferably located immediately downstream of the structural gene.

Examples of a host cell include microorganisms belonging to *Escherichia, Serratia, Bacillus, Brevibacterium, Corynebacterium, Microbacterium, Pseudomonas,* etc. Specific examples of such microorganisms include *Escherichia coli* XL1-Blue, *Escherichia coli* XL2-Blue, *Escherichia coli* BL21, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No.49, *Escherichia coli* W3110, *Escherichia coli* NY49, *Escherichia coli* GI698, *Escherichia coli* TB1, *Serratia ficaria*, *Serratia fonticola, Serratia liquefaciens*, *Serratia marcescens*, *Bacillus subtilis, Bacillus amyloliquefacines, Brevibacterium ammoniagenes, Brevibacterium immariophilum* ATCC14068, *Brevibacterium saccharolyticum* ATCC14066, *Brevibacterium flavum* ATCC14067, *Brevibacterium lactofermentum* ATCC13869, *Corynebacterium glutamicum* ATCC13032, *Corynebacterium glutamicum* ATCC13869, *Corynebacterium acetoacidophilum* ATCC13870, *Microbacterium ammoniaphilum* ATCC15354, *Pseudomonas putida*, and *Pseudomonas* sp. D-0110.

Any method of introducing DNA into the above-described host cell can be used as a method of introducing a recombinant vector therein. Examples of such a method include a method of using calcium ions [Proc. Natl. Acad. Sci. USA, 69, 2110 (1972)], the protoplast method (Japanese Published Unexamined Patent Application No.2483942/1988), and methods described in Gene, 17, 107 (1982) or Molecular & General Genetics, 168, 111 (1979).

In the case of using a yeast strain as a host cell, examples of an expression vector include YEP13 (ATCC37115), YEp24 (ATCC37051), YCp50 (ATCC37419), pHS 19, and pHS 15.

Any promoter can be used as long as it functions in a yeast strain. Examples of such a promoter include a promoter of gene in glycolytic pathway such as hexose kinase, a PHOS promoter, a PGK promoter, a GAP promoter, an ADH promoter, a gal1 promoter, a gal10 promoter, a heat shock polypeptide promoter, an MFα1 promoter, and a CUP1 promoter.

Examples of a host cell include microorganisms belonging to genus of *Saccharomyces, Schizosaccharomyces, Kluyveromyces, Trichosporon, Schwanniomyces, Pichia,* or *Candida*. Specific examples of such microorganisms include *Saccharomyces cerevisiae, Schizosaccharomyces pombe, Kluyveromyces lactis, Trichosporon pullulans, Schwanniomyces alluvius,* and *Candida utilis.*

Any method of introducing DNA into a yeast strain can be used as a method of introducing a recombinant vector. Examples of such a method include the electroporation method [Methods Enzymol., 194, 182 (1990)], the spheroplast method [Proc. Natl. Acad. Sci. USA, 75, 1929 (1978)], the lithium acetate method [J. Bacteriology, 153, 163 (1983)] , and a method described in Proc. Natl. Acad. Sci. USA, 75, 1929 (1978).

In the case of using filamentous fungus as a host cell, examples of an expression vector include pPTRI (manufactured by Hakutsuru Sake Brewing Co., Ltd.), pPTRII (manufactured by Hakutsuru Sake Brewing Co., Ltd.), and pAUR316 (manufactured by Takara Shuzo Co., Ltd.)

Any promoter can be used as long as it functions in filamentous fungi. Examples of such a promoter include an amyB promoter, an enoA promoter, a gpd promoter, and a melO promoter.

Examples of a host cell include *Aspergillus, Penicillium, Tricoderma, Fusarium, Humicola,* and *Mucor* genus.

Any method of introducing DNA into filamentous fungi may be used as a method of introducing a recombinant vector. The protoplast method [GENETICS of ASPERGILLUS NIDULANS: EMBO Practical Course Manual, 8 (1988)] is an example of such a method.

In the case of using an animal cell as a host, examples of an expression vector include pEGFP-C2 (manufactured by Clontech), pAGE107 (Japanese Published Unexamined Patent Application 22979/91; Cytotechnol., 3, 133 1990), pAS3-3 (Japanese Published Unexamined Patent Application No. 227075/90), pCDM8 [Nature, 329, 840 (1987)], pCMV-Tag1 (manufactured by Stratagene), pcDNA3.1(+) (manufactured by Invitrogen), pREP4 (manufactured by Invitrogen), pMSG (manufactured by Amersham Bioscience), and pAMo [J. Biol. Chem., 268, 22782 (1993)].

Any promoter can be used as long as it functions in an animal cell. Examples of such a promoter include a promoter of a cytomegalovirus (CMV) IE (immediate early) gene, an SV40 early promoter, a retrovirus promoter, a metallothionein promoter, a heat shock promoter and an SRα promoter. In addition, an enhancer of a human CMV IE gene may also be used together with a promoter.

Examples of an animal host cell include: Namalwa cell which is derived from human; COS cell which is derived from monkey; CHO cell which is derived from Chinese hamster; and HBT5637 (Japanese Published Unexamined Patent Application No. 299/88).

Any method of introducing DNA into an animal cell can be used as a method of introducing a recombinant vector into an animal cell. Examples of such a method include the electroporation method [Cytotechnology, 3, 133 (1990)], the calcium phosphate method (Japanese Published Unexamined Patent Application No. 227075/90), and the lipofection method [Proc. Natl. Acad. Sci. USA, 84, 7413 (1987), and Virology, 52, 456 (1973)].

In the case of using an insect cell as a host, polypeptides can be expressed, for example, by methods described in Current Protocols in Molecular Biology, John Wiley & Sons (1987), Baculovirus Expression Vectors: A Laboratory Manual, W. H. Freeman and Company (1992), or Bio/Technology, 6, 47 (1988).

That is to say, insect cells are cotransfected with a vector comprising a recombinant gene and baculovirus, so as to obtain a recombinant virus in the culture supernatant of the insect cells. Thereafter, insect cells are infected with the recombinant virus, so that the cells are allowed to express polypeptides.

Examples of a vector for gene transfer used in this method include pVL1392, pVL1393, pBlueBac4.5 (all of which are manufactured by Invitrogen), and pBacPAK9 (manufactured by Clontech).

Autographa califomica nuclear polyhedrosis virus, which is a virus infecting Noctuidae insects, can be used as an example of baculovirus.

Examples of an insect cell include: Sf9 and Sf21, which are ovarian cells of *Spodoptera frugiperda* [Baculovirus Expression Vectors: A Laboratory Manual, W. H. Freeman and Company (1992)]; and High 5, which is ovarian cell of *Trichoplusia ni* (manufactured by Invitrogen).

Examples of a method of cotransfecting insect cells with the above recombinant vector for gene transfer and the above baculovirus, for preparing recombinant virus, include the calcium phosphate method (Japanese Published Unexamined Patent Application No. 227075/90) and the lipofection method [Proc. Natl. Acad. Sci. USA, 84, 7413 (1987)].

In the case of using a plant cell as a host cell, examples of an expression vector include a Ti plasmid and a tobacco mosaic virus vector.

Any promoter can be used as long as it functions in a plant cell. Examples of such a promoter include a cauliflower mosaic virus (CaMV) 35S promoter and a rice actin 1 promoter.

Examples of host cell include plant cells from tobacco, potato, tomato, carrot, soybean, oilseed rape, alfalfa, rice, wheat and barley.

Any method of introducing DNA into plant cells may be used as a method of introducing a recombinant vector. Examples of such a method include the Agrobacterium method (Japanese Published Unexamined Patent Application Nos. 140885/84 and 70080/85, and WO94/00977), the electroporation method (Japanese Published Unexamined Patent Application No. 251887/85), and a method of using a particle gun (Japanese Patent Nos. 2606856 and 2517813).

The transformant of the present invention obtained as above is cultured in a medium, and the polypeptide of the present invention is produced and accumulated in the obtained culture, and then, it is recovered from the culture. Thus, the polypeptide of the present invention can be produced.

The transformant of the present invention can be cultured by a conventional method used in the culture of a host.

Where the transformant of the present invention is obtained using, as a host, a prokaryote such as *Escherichia coli* or eukaryote such as a yeast or filamentous fungus, a medium in which the transformant is cultured may be either a natural medium or synthetic medium, as long as it contains a carbon source that can be assimilated by the transformant, nitrogen source or inorganic salts, and the transformant can be efficiently cultured therein.

Any carbon source can be used as long as it can be assimilated by the transformant. Examples of such a carbon source include carbonhydrates such as glucose, fractose, sucrose, molasses containing these carbonhydrates, starch or starch hydrolysate, organic acids such as acetic acid or propionic acid, and alcohols such as ethanol or propanol.

Examples of a nitrogen source used herein include ammonium salts of inorganic or organic acids such as ammonia, ammonium chloride, ammonium sulfate, ammonium acetate or ammonium phosphate, other nitrogen-containing compounds, peptone, meat extract, yeast extract, corn steep liquor, casein hydrolysate, wheat protein, wheat protein hydrolysate, soybean cake, soybean cake hydrolysate, various types of fermentative bacteria, and digests thereof.

Examples of an inorganic salt used herein include potassium dihydrogenphosphate, dipotassium hydrogenphosphate, magnesium phosphate, magnesium sulfate, sodium chloride, ferrous sulfate, manganese sulfate, cupric sulfate, and calcium carbonate.

Culturing is carried out under aerobic conditions such as shaking culture or submerged spinner culture under aeration. The culture temperature is generally between 15°C and 40°C, and the culture time is generally between 16 hours and 7 days. The pH for culture is preferably maintained between 3.0 and 9.0. The pH is adjusted using inorganic or organic acid, an alkali solution, urea, calcium carbonate, ammonia, etc.

In addition, antibiotics such as ampicillin or tetracycline may be added to the medium during the culture, as necessary.

In the case of culturing microorganisms transformed with a recombinant vector comprising an inducible promoter, an inducer may be added to a medium, as necessary. For example, in the case of culturing microorganisms transformed with a recombinant vector comprising a *lac* promoter, isopropyl-β-D-thiogalactopyranoside may be added to a medium. In the case of culturing microorganisms transformed with a recombinant vector comprising a *trp* promoter, indoleacrylic acid may be added to a medium.

Examples of a medium in which a transformant obtained from an animal cell as a host is cultured include RPMI 1640 medium [J. Am. Med. Assoc., 199, 519 (1967)], Eagle's MEM (minimum essential medium) [Science, 122, 501 (1952)], Dalbecco's modified Eagle's medium [Virology, 8, 396 (1959)], and 199 medium [Proc. Soc. Exp. Biol. Med., 73, 1 (1950)], which are commonly used, and a medium obtained by adding fetal bovine serum or the like to these media.

The culturing is usually carried out at pH 6 to 8 at 30 to 40°C for 1 to 7 days in the presence of 5% CO₂.

In addition, antibiotics such as kanamycin or penicillin may also be added to the medium during the culturing, as necessary.

Examples of a medium in which a transformant obtained from an insect cell as a host is cultured include TNM-FH medium (manufactured by Pharmingen), Sf-900 II SFM medium (manufactured by Invitrogen), ExCel1400 and ExCel1405 (both of which are manufactured by JRH Bioscience), and Grace's insect medium [Nature, 195, 788 (1962)], which are commonly used.

The culturing is usually carried out at pH 6 to 7 at 25 to 30°C for 1 to 5 days.

In addition, antibiotics such as gentamicin may also be added to the medium during the culturing, as necessary.

The transformant obtained from a plant cell as a host can be cultured directly or after differentiating into plant cells or organs. Examples of a medium in which the transformant is cultured include Murashige and Skoog (MS) medium and White medium, which are commonly used, and a medium obtained by adding plant hormone such as auxin or cytokinin to these mediums.

The culturing is usually carried out at pH 5 to 9 at 20 to 40°C for 3 to 60 days.

In addition, antibiotics such as kanamycin or hygromycin may also be added to the medium during the culturing, as necessary.

As stated above, a transformant derived from microorganisms, animal cells or plant cells comprising a recombinant vector into which DNA encoding the polypeptide of the present invention is incorporated is cultured by an ordinary culturing method, the polypeptide is produced and accumulated in the culture, and the polypeptide is then recovered from the culture. Thus, the polypeptide of the present invention can be produced.

When the polypeptide of the present invention is expressed using yeast strains, filamentous fungi, animal cells, insect cells or plant cells, a polypeptide to which sugar or sugar chain is added can be obtained.

Processes for producing the polypeptide of the present invention include a method of allowing a host cell to produce it inside the cell, a method of allowing a host cell to secrete it out of the cell, and a method of allowing a host cell to produce it on an outer membrane thereof. Such a method can be selected depending on the type of a host cell to be used or the type of the structure of a polypeptide to be produced.

Even where the polypeptide of the present invention is produced inside a host cell or on the outer membrane of the host cell, it is possible to actively secrete the polypeptide out of the host cell by applying the method of Paulson et al. [J. Biol. Chem., 264, 17619 (1989)], the method of Lowe et al. [Proc. Natl. Acad. Sci. USA, 86, 8227 (1989), Genes Develop., 4, 1288 (1990)], or the method described in Japanese Published Unexamined Patent Application No. 336963/1993, WO94/23021, and the like.

That is to say, the polypeptide of the present invention is expressed by gene recombination such that a signal peptide is added just before a polypeptide comprising an active site of the polypeptide of the present invention, so that the polypeptide of the present invention can be actively secreted out of a host cell.

Moreover, the production amount of polypeptide can be increased by applying the method described in Japanese Published Unexamined Patent Application No. 227075/1990, using a gene amplification system comprising a dihydrofolate reductase gene or the like.

Furthermore, the polypeptide of the present invention can be produced by applying the known method [J. Biomol. NMR, 6, 129 (1998), Science, 242, 1162 (1988), J. Biochem., 110, 166 (1991)], using an *in vitro* transcription and translation system. This is to say, DNA encoding the polypeptide of the present invention is ligated downstream of a promoter such as SP6, T7 or T3, and it is then reacted with RNA polymerase specific for such a promoter, so that a large amount of RNA encoding the polypeptide of the present invention is synthesized *in vitro.* Thereafter, the polypeptide of the present invention can be produced using a cell-free translation system such as a translation system using a rabbit reticulocyte lysate or wheat germ extract.

In order to isolate and purify the polypeptide produced by the transformant of the present invention, an ordinary method of isolating and purifying enzymes can be used. For example, where the polypeptide of the present invention is expressed in a state dissolved in cells, after completion of the culturing, cells are recovered by centrifugal separation, and the recovered cells are suspended in a water-based buffer. Thereafter, the cells are disintegrated using an ultrasonic disintegrator, French press, Manton Gaulin homogenizer or Dyno mill, so as to obtain a cell-free extract. A supernatant is obtained by centrifuging the cell-free extract, and then, a purified sample can be obtained from the supernatant by applying, singly or in combination, the following ordinary enzyme isolation and purification methods: the solvent extraction, the salting-out method using ammonium sulfate, the desalting method, the precipitation method using an organic solvent, the anion exchange chromatography using resins such as diethylaminoethyl (DEAE) sepharose or DIAION HPA-75 (manufactured by Mitsubishi Chemical Corp.), the cation exchange chromatography using resins such as S-Sepharose FF (manufactured by Pharmacia), the hydrophobic chromatography using resins such as butyl sepharose or phenyl sepharose, the gel filtration method using a molecular sieve, the affinity chromatography, the chromatofocusing method, and the electrophoresis such as isoelectric focusing. Where the above polypeptide is tagged and then expressed using a pRSET vector (manufactured by Invitrogen) or pGEX vector (manufactured by Amersham Bioscience), affinity purification can be carried out by using an appropriate carrier such as a nickel resin or glutathione sepharose.

Where the above polypeptide is expressed with formation of an insoluble form in cells, the cells are recovered, disintegrated and centrifuged in the same manner as described above, so that the insoluble form of polypeptide is recovered as a precipitation fraction. Thereafter, the insoluble form of the recovered polypeptide is solubilized using a polypeptide denaturing agent. The obtained solubilized solution is diluted or dialyzed, so that the concentration of the polypeptide denaturing agent contained in the above solubilized solution is decreased. The polypeptide is thus returned to a normal three-dimensional structure. After this operation is performed, the same above isolation and purification method is applied to the obtained polypeptide, so as to obtain a purified sample.

Where the polypeptide of the present invention or a derivative thereof such as a polypeptide obtained by adding a sugar chain to the above polypeptide is extracellularly secreted, the above polypeptide or a derivative thereof can be recovered from a culture supernatant. This is to say, the culture is treated by the same above techniques such as centrifugal separation, so as to obtain a culture supernatant. Thereafter, a purified sample can be obtained from the culture supernatant by the isolation and purification method as mentioned above.

Examples of the polypeptide of the present invention obtained as above include a polypeptide comprising an amino acid sequence shown in SEQ ID NO: 2 and a polypeptide comprising an amino acid sequence shown in SEQ ID NO: 10.

Moreover, the polypeptide of the present invention can be produced by chemical synthesis methods such as the Fmoc method (fluorenylmethyloxycarbonyl method) or the tBoc method (t-butyloxycarbonyl method). Furthermore, the polypeptide of the present invention can also be synthesized using peptide synthesizers that are available from Applied Biosystems, Advanced ChemTech, Shimadzu Corporation, etc.

Still further, the polypeptide of the present invention can also be produced by culturing a microorganism having an ability of producing the polypeptide of the present invention. Any microorganism can be used as long as it has an ability of producing the polypeptide of the present invention. Preferred examples of such a microorganism include filamentous fungus. More preferred examples include filamentous fungus belonging to a genus selected from a group consisting of *Aspergillus, Penicillium, Humicola, Trichoderma, Mucor,* and *Fusarium.* Further more preferred example include filamentous fungus belonging to *Aspergillus,* such as filamentous fungus belonging to a species selected from a group consisting of *Aspergillus oryzae, Aspergillus sojae, Aspergillus niger, Aspergillus awamori, Aspergillus kawachii,* *Aspergillus parasiticus, Aspergillus flavus, Aspergillus nomius, Aspergillus fumigatus,* and *Aspergillus nidulans*. The microorganism having an ability of producing the polypeptide of the present invention may be any of a wild strain, a transformant and a mutant, but it is preferably a microorganism that has been subjected to transformation, mutation or the like and thereby has an increased ability of producing the polypeptide of the present invention. Examples of such mutation treatment include ultraviolet irradiation and treatment with mutagenic agents such as N-methyl-N'-nitro-N-nitrosoguanidine. Culturing of the microorganism and purification of the polypeptide can be carried out in the same manner as in the above-described culturing of a transformed microorganism and purification of a polypeptide.

### (3) Process for producing protein hydrolysate

The polypeptide of the present invention having a pyroglutamyl peptidase activity and protein hydrolase are added to a raw material containing a protein, and the mixture is blended, and then reaction is carried out generally between 20°C and 60°C, preferably between 30°C and 50°C, for 24 to 264 hours, preferably for 48 to 240 hours, so as to produce a protein hydrolysate. Otherwise, protein hydrolase is first added to a raw material containing a protein, and the mixture is blended, and then reaction is carried out generally between 20°C and 60°C, preferably between 30°C and 50°C, for 24 to 264 hours, preferably for 48 to 240 hours for the hydrolysis of the protein. Thereafter, the polypeptide of the present invention having a pyroglutamyl peptidase activity is added to the reaction product, and the mixture is blended, and then reaction is carried out generally between 20°C and 60°C, preferably between 30°C and 50°C, for 5 to 96 hours, preferably for 12 to 72 hours, so as to produce a protein hydrolysate. In the latter case, when the polypeptide of the present invention having a pyroglutamyl peptidase activity is added to the reaction product, protein hydrolase may also be added thereto. During the reaction, any pH value may be applied as long as the polypeptide of the present invention having a pyroglutamyl peptidase activity and protein hydrolase can act in the pH. The pH is preferably adjusted to pH 5 to 8.

As a polypeptide having a pyroglutamyl peptidase activity used in this production method, a polypeptide purified by the method described in (2) above can be used. Alternatively, a polypeptide may not be purified, and a culture product or treated product thereof containing the polypeptide of the present invention having a pyroglutamyl peptidase activity, which is obtained by culturing in a medium the above-described transformant containing recombinant DNA comprising DNA encoding the polypeptide of the present invention having a pyroglutamyl peptidase activity described in (2) above, or microorganism having an ability of producing the polypeptide of the present invention having a pyroglutamyl peptidase activity, may also be used.

Examples of protein hydrolase include Flavorzyme (manufactured by Novo Nordisk) and a culture product of Koji molds. Any type of Koji molds may be used as long as it is used in the brewing industry. Examples of such Koji molds include *Aspergillus oryzae* and *Aspergillus sojae.* The type of a protein contained in a raw material used in the production method of the present invention is not particularly limited. A protein containing a large amount of glutamic acid is preferable. Moreover, any type of raw material may be used as long as it contains a large amount of protein. It is not necessarily a purified protein. Examples of such a material include wheat gluten, defatted soybeans, and a separated soybean protein. After completion of the reaction, unreacted raw material protein, fungus bodies or the like are removed out, and the supernatant is concentrated and dried, as necessary, so that a protein hydrolysate at a high hydrolysis rate can be obtained.

### (4) Production of antibody which specifically recognizes the polypeptide of the present invention

### (a) Production of polyclonal antibody

A polyclonal antibody which specifically binds to the polypeptide of the present invention can be produced by immunization of animals, using, as an antigen, a purified sample from the full-length polypeptide of the present invention obtained by the method described in (2) above or a partial fragment thereof, or a peptide having an amino acid sequence of a part of the peptide of the present invention. As an immunization method, an antigen may be directly administered into the subcutis, vein or abdominal cavity of an animal, but it is preferable to bind a highly antigenic carrier protein with an antigen for administration, or to administer an antigen together with a suitable adjuvant.

A peptide used as an antigen can be chemically synthesized by applying chemical synthesis methods such as the Fmoc method (fluorenylmethyloxycarbonyl method) or the tBoc method (t-butyloxycarbonyl method), or using peptide synthesizers that are available from Applied Biosystems, Advanced ChemTech, Shimadzu Corporation, etc.

Examples of a carrier protein include keyhole limpet hemocyanin, bovine serum albumin, and bovine thyroglobulin. Examples of an adjuvant include Complete Freund's Adjuvant, aluminum hydroxide gel, and pertussis vaccine.

Examples of an animal to be immunized include non-human mammals such as a rabbit, goat, mouse, rat or hamster.

Antigen is administered 3 to 10 times every 1 to 2 weeks after the first administration. On the third to seventh day after completion of each administration, a blood sample is collected from the venous plexus of the fundus oculi to prepare serum. Thereafter, it is confirmed by enzyme linked immunoassay (ELISA) [Koso Meneki Sokutei Ho (Enzyme linked Immunoassay) 3^{rd} edition, Igaku-Shoin Ltd. (1987); Antibodies: A Laboratory Manual, Cold Spring Harbor Laboratory Press (1988)] or the like that the prepared serum is reacted with the antigen that has been used for the immunization. The dosage of the antigen is preferably 50 to 200 µg per animal per administration.

The total serum is obtained from animals whose serum shows a sufficient antibody titer to the antigen used in immunization, and the obtained serum is then separated and purified, so as to obtain a polyclonal antibody. Examples of such separation and purification methods include centrifugal separation, salting-out using 40% to 50% saturated ammonium sulfate, caprylic acid precipitation [Antibodies: A Laboratory Manual, Cold Spring Harbor Laboratory Press, (1988)], or methods in which chromatographies using a DEAE-sepharose column, anion exchange column, protein A or G column, or gel filtration column are used singly or in combination.

### (b) Production of monoclonal antibody

### (i) Preparation of antibody-producing cells

A mouse or rat whose serum shows a sufficient antibody titer to the antigen used in immunization in (a) above is used as a source for supplying antibody-producing cells.

On the third to seventh day after the final administration of an antigenic substance to the mouse or rat whose serum shows a sufficient antibody titer, the spleen is extirpated therefrom. The spleen is cut finely in MEM (Minimum Essential Medium), and is taken apart to pieces with tweezers, followed by performing centrifugal separation thereon at 1,200 rpm for 5 minutes. Thereafter, the obtained supernatant is discarded. Spleen cells contained in the thus obtained precipitation fractions are treated with Tris-ammonium chloride buffer solution (pH 7.65) for 1 to 2 minutes, so as to eliminate erythrocytes. Thereafter, the residue is washed with MEM 3 times. The obtained spleen cells are used as antibody-producing cells.

### (ii) Preparation of myeloma cells

A cell line established from a mouse or rat is used as myeloma cells. For example, 8-azaguanine resistant mouse (BALB/c derived) myeloma cell lines such as P3-X63Ag8-U1 [Curr. Topics Microbiol. Immunol., 81, 1 (1978), Eur. J. Immunol., 6, 511 (1976)], SP2/0-Ag14 [Nature, 276, 269 (1978)], P3-X63-Ag8653 [J. Immunol., 123, 1548 (1979)], or P3-X63-Ag8 [Nature, 256, 495 (1975)] can be used. These cell lines are subcultured in a 8-azaguanine medium [obtained by adding 1.5 mmol/l glutamine, 50 µmol/l 2-mercaptoethanol, 10 µg/ml gentamicin and 10% fetal bovine serum to an RPMI 1640 medium (hereinafter referred to as a normal medium), and further adding 15 µg/ml 8-azaguanine thereto]. The cell lines are cultured in a normal medium 3 to 4 days before cell fusion, and the 2 x 10⁷ cells are used for the fusion.

### (iii) Preparation of hybridomas

The antibody-producing cells obtained in (i) above and the myeloma cells obtained in (ii) above are well washed with MEM or PBS (1.83 g/l disodium phosphate, 0.21 g/l monopotassium phosphate, 7.65 g/l NaCl, pH 7.2), and these cells are mixed such that the number of cells becomes the antibody-producing cells : the myeloma cells = 5 to 10 : 1. The mixture is then centrifuged at 1,200 rpm for 5 minutes, and the obtained supernatant is discarded.

A mass of cells contained in the obtained precipitation fractions is well loosen. Thereafter, to the cells, 0.2 to 1 ml of a solution obtained by mixing 2 g of polyethylene glycol-1000, 2 ml of MEM and 0.7 ml of dimethyl sulfoxide is added per 108 antibody-producing cells at 37°C, while stirring. Thereafter, 1 to 2 ml of MEM is further added thereto several times every 1 or 2 minutes. After completion of the addition, MEM is added to the obtained mixture to the total amount of 50 ml. The thus prepared solution is centrifuged at 900 rpm for 5 minutes, and the obtained supernatant is then discarded.

Cells contained in the obtained precipitation fractions are gently taken apart to pieces, and the cells are gently suspended in 100 ml of an HAT medium [obtained by adding 0.4 mmol/l hypoxanthine, 15 µmol/l thymidine and 0.4 µmol/l aminopterin to a normal medium] by sucking and blowing with a measuring pipet. The suspension is poured into each hole of a 96-hole culture plate in an amount of 100 µl/hole, and it is then cultured at 37°C for 7 to 14 days in a 5% CO₂ incubator.

After completion of the culturing, an aliquot is taken from the culture supernatant, and antibodies binding to the polypeptide of the present invention are detected in the culture supernatant by ELISA, so as to select hybridomas which produce the monoclonal antibodies specifically binding to the polypeptide of the present invention.

The following method may be a specific example of ELISA. A polypeptide or peptide used as an antigen in immunization is coated on an appropriate plate. It is reacted with the hybridoma culture supernatant or a purified antibody obtained in (iv) described below as a primary antibody. Thereafter, it is reacted with an anti-mouse immunoglobulin antibody (in a case where antibody-producing cells are derived from a rat, then an anti-rat immunoglobulin antibody is used) labeled with an enzyme such as horseradish peroxidase, as a secondary antibody. A substrate that develops color by a labeling enzyme is added to the above reaction product, and reaction is carried out, so that a primary antibody binding to the antigen is detected.

The above hybridomas are cloned twice by limiting dilution method [for the first cloning, an HT medium (obtained by eliminating aminopterin from the HAT medium) is used, and for the second cloning, a normal medium is used]. Hybridomas which stably show a high antibody titer are selected as a hybridoma cell line which specifically binds to the polypeptide of the present invention.

### (iv) Preparation of monoclonal antibodies

The hybridoma cells obtained in (iii) above that produce a monoclonal antibody specifically binding to the polypeptide of the present invention are injected in an amount of 5 to 20 x 10⁶ cells/mouse into the abdominal cavity of 8 to 10 week old mice or nude mice which are previously administered intraperitoneally with 0.5 ml of pristane (2,6,10,14-tetramethylpentadecane) and then grown for 2 weeks. 10 to 21 days later, the hybridomas turn cancerous in the ascites. The ascites is collected from the mice with the thus cancerous ascites, and it is then centrifuged at 3,000 rpm for 5 minutes to eliminate solids. Monoclonal antibodies can be purified and obtained from the obtained supernatant by the same method as applied in the purification of polyclonal antibodies.

The subclass of antibodies is determined using a mouse monoclonal antibody typing kit or rat monoclonal antibody typing kit. The amount of protein is determined by the Lowry method, or it is calculated from an absorbance at 280 nm.

### (5) Detection and quantification of polypeptide of the present invention using antibody specifically binding to polypeptide of the present invention

Using the antibody obtained in (4) above which specifically binds to the polypeptide of the present invention, an antigen-antibody reaction is carried out, so that the polypeptide of the present invention can be immunologically detected and quantified. As a measurement sample, a cell extract, a culture supernatant and the like is used.

Examples of an immunological detection and quantification method include the fluorescent antibody technique, ELISA, the radioimmunoassay (RIA), the immunohistochemistry or immunocytochemistry, the immunoblotting, the dot blotting, the immunoprecipitation method, and the sandwich ELISA [Tan Clone Kotai Jikken Manual (Monoclonal Antibody Experimental Manual) (Kodansha Scientific) (1987), Zoku Seikagaku Jikken Koza 5 (Follow-Up Biochemical Experiment Seminars 5); Meneki Seikagaku Kenkyu Ho (Research Methods of Immunobiochemistry) (Tokyo Kagaku Dojin Co., Ltd.) (1986)].

The fluorescent antibody technique is a method involving reacting a measurement sample with an antibody specifically binding to the polypeptide of the present invention, then reacting the measurement sample with an antibody binding to the above antibody (e.g, in a case where the antibody specifically binding to the polypeptide of the present invention is a mouse antibody, it is an anti-mouse IgG antibody or fragment thereof), which has been labeled with a fluorescent substance such as fluorescin isocyanate (FITC), and measuring the fluorescent dye with a flow cytometer, so as to detect the polypeptide of the present invention.

The ELISA is a method involving reacting a measurement sample with an antibody specifically binding to the polypeptide of the present invention, then reacting the measurement sample with an antibody binding to the above antibody, which has been labeled with an enzyme such as peroxidase, adding thereto a substrate that develops color by addition of a labeling enzyme, performing reaction, and measuring a coloring dye with a spectrophotometer, so as to detect the polypeptide of the present invention.

The RIA is a method involving reacting a measurement sample with an antibody specifically binding to the polypeptide of the present invention, then reacting the measurement sample with an antibody binding to the above antibody, which has been labeled with a radioactive label, and measuring radioactivity with a scintillation counter or the like, so as to detect the polypeptide of the present invention.

The immunohistochemistry and immunocytochemistry is a method involving reacting a measurement sample such as a cell or tissue section with an antibody specifically binding to the polypeptide of the present invention, then reacting the measurement sample with an antibody binding to the above antibody, which has been labeled with a fluorescent substance such as FITC, peroxidase or biotin, and observing the sample with a microscope, so as to detect the polypeptide of the present invention.

The immunoblotting (Western blotting) is a method involving fractionating a measurement sample by SDS-PAGE [Antibodies- A Laboratory Manual, Cold Spring Harbor Laboratory, (1988)], blotting the gel onto a PVDF membrane or nitrocellulose membrane, reacting the membrane with an antibody specifically binding to the polypeptide of the present invention, then reacting it with an antibody binding to the above antibody, which has been labeled with a fluorescent substance such as FITC, peroxidase or biotin, and carrying out a reaction depending on the labeling substance, so as to detect the polypeptide of the present invention.

The dot blotting is a method involving blotting a measurement sample onto a nitrocellulose membrane, reacting the membrane with an antibody specifically binding to the polypeptide of the present invention, then reacting it with an antibody binding to the above antibody, which has been labeled with a fluorescent substance such as FITC, peroxidase or biotin, and carrying out a reaction depending on the labeling substance, so as to detect the polypeptide of the present invention.

The immunoprecipitation method is a method involving reacting a measurement sample with an antibody specifically binding to the polypeptide of the present invention, and adding thereto a carrier having an ability of specifically binding to immunoglobulin, such as protein A-sepharose, to form an antigen-antibody complex, followed by separation, so as to detect the polypeptide of the present invention.

The sandwich ELISA is a method involving reacting a measurement sample with a plate onto which an antibody specifically binding to the polypeptide of the present invention is adsorbed, then reacting the measurement sample with an antibody specifically binding to the polypeptide of the present invention and having an antigen recognition site different from that of the above antibody, which has been labeled with an enzyme such as peroxidase, adding thereto a substrate that develops color by a labeled enzyme, and measuring a coloring dye with a spectrophotometer, so as to detect the polypeptide of the present invention.

A solution containing a certain concentration of a purified sample of the polypeptide of the present invention which can be prepared by the method described in (2) above is prepared, and the solution is diluted stepwise to obtain sample dilutions. These samples are measured by the above detection methods. A calibration curve is prepared from the measurement values of samples with different concentrations. Thus, the polypeptide of the present invention can be quantified by comparing the measurement values of measurement samples.

The present invention will be further specifically described in the following examples, but the scope of the present invention is not limited thereby. Various gene manipulations described in the examples were carried out in accordance with methods described in Current Protocols in Molecular Biology, John Wiley & Sons (1987).

### BEST MODE FOR CARRYING OUT THE INVENTION

### Example 1 Method of producing whole genome shot gun library

### 1. Production of insert DNA

### (1) Obtainment f chromosomal DNA

Spores of filamentous fungus *Aspergillus oryzae* RIB 40 (ATCC No. 42149) were inoculated into a YPD medium (0.5% yeast extract, 1% peptone, 2% glucose), and the mixture was subjected to a shaking culturing at 30°C overnight. Thereafter, genomic DNA was extracted in accordance with the method of Iimura [Agric. Biol. Chem., 51, 323 (1987)]. Mitochondrial DNA that had existed with the genomic DNA was purified by cesium chloride ultracentrifugation in accordance with the method of Watson et al. [Methods Enzymol., 118, 57 (1986)], so as to obtain only chromosomal DNA.

### (2) Fragmentation of chromosomal DNA

Using a random DNA fragmentation device HydroShear (Tomy Seiko Co., Ltd.), the obtained pure chromosomal DNA was degraded into fragments with a size of approximately 1 to 2 kb.

### (3) Treatment of fragmented DNA termini

The fragmented chromosomal DNA was treated with BAL31 nuclease (Takara Shuzo Co., Ltd.) and then treated with a Klenow fragment (Takara Shuzo Co., Ltd.), so that the termini were blunted.

### (4) Addition of adaptor to termini

DNA fragments comprising nucleotide sequences shown in SEQ ID NOS: 6 and 7, 5' termini of which had been phosphorylated, were used as adaptors. The adaptors were ligated to both termini of the blunt-ended chromosomal DNA fragment, using T4 DNA ligase (Takara Shuzo Co., Ltd.).

### 2. Insertion of insert DNA into vector and transformation

pUC19 was cleaved with a restriction enzyme *Sal*I (Takara Shuzo Co., Ltd.), and a thymidine residue was then inserted into the *Sal*I cleavage site with Taq DNA polymerase (Roche Diagnostics). The thus prepared plasmid was treated with alkaline phosphatase (Takara Shuzo Co., Ltd.) for dephosphorylation, and it was then used as a vector. The vector was ligated to the above-prepared chromosomal DNA fragment using T4 DNA ligase, and then, *Escherichia coli* DH10B (Gibco) was transformed therewith by the electroporation.

### 3. Nucleotide sequencing

The transformed *Escherichia coli* was cultured in a 2 x YP medium at 37°C for 10 hours. After collecting cell bodies, they were subjected to heat treatment in a sterilized water at 99°C for 10 minutes. The obtained supernatant was used as a template DNA aqueous solution. The full length insert fragment containing a region to be annealed with a primer for sequencing was amplified by PCR consisting of 30 cycles of 20 seconds at 98°C and 2 minutes at 68°C. The obtained DNA fragment was used as a template for the Sanger method. An M13 Universal primer or M13 Reverse primer and a PRISM Dye-Terminator sequencing kit manufactured by Perkin Elmer were used, and a sequencing reaction was carried out in accordance with an instruction manual attached to the kit. An unreacted Dye-terminator was eliminated from the sequencing reaction product by the gel filtration method or the like. Thereafter, the nucleotide sequence of the DNA fragment was determined using a 3700 DNA Sequencer manufactured by Perkin Elmer. The waveform data outputted from the 3700 DNA Sequencer were reanalyzed with Phred (Phil Green). The vector and the adaptor sequence were eliminated, and the remaining sequences were assembled using SPS Phrap (Southwest Parallel Software), so as to construct contigs of the nucleotide sequence of the genomic DNA of *Aspergillus oryzae* RIB 40.

### Example 2 Identification of gene

Identification of gene from the nucleotide sequence of the genomic DNA was carried out using, in combination, a gene finding system GeneDecoder based on the algorithm according to Kiyoshi Asai et al. [Pacific Symposium on Biocomputing, 98, 228 (1998)] and a gene finding system ALN based on the algorithm according to Osamu Goto [Bioinformatics, 16, 190-202 (2000)] to the contigs of the nucleotide sequence of the genomic DNA, while taking into consideration the EST sequence information that had already been obtained, and information regarding homology to the known protein amino acid sequence database, by applying the methods according to the following (1) to (7).

### (1) Extraction of BLAST homologous gene candidate regions

A region having a high homology with the amino acid sequence of a known protein is extracted from the contigs of the nucleotide sequence of the genomic DNA. The homology of an amino acid sequence can be determined by the algorithm BLAST according to Karlin and Altschul [Proc. Natl. Acad. Sci. USA, 87, 2264 (1990), Proc. Natl. Acad. Sei. USA, 90, 5873 (1993)]. A program called BLASTX had been developed based on this algorithm [J. Mol. Biol., 215, 403-410, (1990)], and when the nucleotide sequence of genomic DNA is translated into an amino acid sequence, a region with high homology can be directly searched by the program. Specific techniques of these analysis methods are known (http://www.ncbi.nlm.nih.gov). In this technique, SWISSPROT version 39 [Nucleic Acids Res., 28, 45 (2000)] and NRaa are searched as database by BLASTX using contig of the nucleotide sequence of genomic DNA as query. Thus, regions in which an index of homology in the BLAST algorithm, E-value, is 10⁻³⁰ or lower (the lower the E-value, the higher the homology is) are extracted. Gene candidate regions obtained by preferentially extracting regions with higher homology from the above regions without overlapping with one another were defined as BLAST homologous gene candidate regions.

### (2) Extraction of ALN gene candidate regions

The gene finding system ALN was applied to contig sequences to select candidate regions of homologous genes extracted by BLAST, using a region having homology to a region corresponding to 90% or more of the full-length amino acid sequence of a protein that is a target of homology as a core. The extracted gene candidate region was defined as an ALN gene candidate region. The ALN specifies a splice site by making the full-length amino acid sequence of a protein that is a target of homology align against the contigs, so as to predict gene regions.

### (3) Extraction of GD homologous gene candidate regions

The gene finding system GeneDecoder was applied to contig sequences to select candidate regions of homologous genes extracted by BLAST, using a region having homology to a region corresponding to 20% or more to less than 90% of the length of residues of the amino acid sequence of a protein that is a target of homology as a core. The extracted gene candidate region was defined as a GD homologous gene candidate region. The GeneDecoder integrates the E-value of BLASTX with the dicodon bigram values that is an index of the orientation of a protein encoding region, and further takes into consideration the position-dependent 1^{st} order Markov model score of a splice site, so as to predict gene regions.

### (4) Extraction of EST-GD gene candidate regions

With regard to regions in which gene expression has been confirmed by EST corresponding to contig sequences, the GeneDecoder is applied to contig sequences around the regions. Thus, not only gene regions determined by the sequences of EST, but also gene candidate regions extracted by predicting the entire gene regions, are defined as EST-GD gene candidate regions.

### (5) Extraction of general GD gene candidate regions

The GeneDecoder is applied to contig sequences that are not contained in the gene candidate regions defined in (1) to (4) above. Thus, gene candidate regions extracted by predicting gene regions are defined as general GD gene candidate regions.

### (6) Extraction of tRNA gene candidate regions

A tRNA-scan is applied to all the contigs, and the extracted tRNA gene candidates are defined as tRNA gene candidate regions.

### (7) Integration of gene candidate regions

The gene candidate regions defined in (2) to (6) are integrated by the following procedure: First, from the gene candidate regions defined in (2) to (6), those predicting gene regions which contradict splice sites determined by EST, are eliminated. The remaining gene candidate regions are integrated by removing those overlapping with each other. In this operation, the gene candidate regions are integrated preferentially in the order of the tRNA gene candidate regions, the ALN homologous gene candidate regions, the GD homologous gene candidate regions, the GD-EST gene candidate regions, and the general GD gene candidate regions. The thus integrated gene candidate region is defined as a set of predicted genes. The nucleotide sequence shown in SEQ ID NO: 1 is one of the thus obtained predicted genes.

By the above procedure, in terms of homology, it is assured that a gene having homology to the full length of a known protein, a gene having a certain level of homology to the known protein, and a gene having no homology to the known protein are specified with reliability in this order. Moreover, in terms of confirmation of expression, genes are specified with reliability in the order of a gene whose expression has been confirmed by EST and a gene whose expression has not been confirmed by EST. Furthermore, it is assumed that all the candidate genes do not contradict splice sites specified by EST.

All the used techniques adopts algorithm that does not allow a termination codon to be contained in a protein encoding region, and accordingly, it is unlikely to predict a pseudogene as a gene of interest.

The functions of the predicted gene region were determined by performing homology search against database Nraa using BLAST, and setting homology sufficient to specify the functions (an E-value of 10⁻³⁰) as a threshold value.

Furthermore, in order to extract pyroglutamyl peptidase from these predicted genes with high precision, the nucleotide sequence of a known pyroglutamyl peptidase gene was searched using the BLASTX against database including query and the above predicted gene set. As a result, it was found that human pyroglutamyl peptidase I (GenBank registration No. AJ278828) and a predicted gene comprising the nucleotide sequence shown in SEQ ID NO: 1 had homology with each other, showing an E-value of 6.1 x 10⁻⁵. Accordingly, it was considered that DNA comprising the nucleotide sequence shown in SEQ ID NO: 1 encodes pyroglutamyl peptidase derived from *Aspergillus oryzae.* In addition, it was also considered that an intron exists in a portion of the nucleotide sequence shown in SEQ ID NO: 1 and that the intron encodes the amino acid sequence shown in SEQ ID NO: 2. The nucleotide sequence of a 5' non-translation region of the nucleotide sequence shown in SEQ ID NO: 1, which is considered to contain a region to function as a promoter, was shown in SEQ ID NO: 3. Likewise, the nucleotide sequence of a 3' non-translation region thereof was shown in SEQ ID NO: 4. Moreover, the nucleotide sequence of a coding region from which the sequences of the intron and the non-translation regions are eliminated was shown in SEQ ID NO: 5.

### Example 3 Cloning of pyroglutamyl peptidase cDNA of Aspergillus oryzae

### (1) Preparation of cDNA of Aspergillus oryzae RIB 40 strain

*Aspergillus oryzae* RIB 40 strain was cultured under the following conditions. This is to say, this strain was inoculated into 60 ml of a DPY medium (2% dextrin, 2% polypeptone, 0.5% yeast extract, 0.5% monopotassium phosphate, 0.05% magnesium sulfate 7 hydrates), and the mixture was placed in a 300 ml Erlenmeyer flask with a baffle, followed by a shaking culturing at 30°C at 150 rpm for 2 days. Thereafter, the culture was filtrated, and 1 g of the obtained wet cell bodies was transferred into a mortar containing liquid nitrogen, so that the cell bodies were frozen by the liquid nitrogen. Thereafter, the cell bodies were ground with a pestle into fine powders.

Total RNA was obtained from this fine powders, using an RNeasy Midi Kit manufactured by Qiagen.

From the obtained total RNA, 100 µl of 0.6 µg/ml mRNA solution was obtained, using an Oligotex™-dT30 <Super> mRNA Purification Kit manufactured by Takara Shuzo Co., Ltd. 10 µl of 3 mol/l sodium acetate solution (pH 5.2) and 250 µl of 99.5% ethanol were added to this solution. The obtained mixture was intensively stirred, and it was then left at rest at -20°C for 2 hours. Thereafter, the mixture was centrifuged at 12000 rpm for 20 minutes, and the precipitate was then washed with 200 µl of 70% ethanol. Thereafter, it was dissolved in 6 µl of a diethylpyrocarbonate-treated water.

The first strand cDNA and the second strand cDNA were synthesized from the recovered mRNA using a SUPERSCRIPT Plasmid System with GATEWAY™ Technology for cDNA Synthesis and Cloning manufactured by Invitrogen, and they were used as templates for PCR.

### (2) Cloning of pyroglutamyl peptidase cDNA of Aspergillus oryzae by PCR, and construction of plasmid used for expression of pyroglutamyl peptidase

Primers comprising nucleotide sequences shown in SEQ ID NOS: 8 and 9 were designed from the nucleotide sequence shown in SEQ ID NO: 1, and were synthesized.

Using the above primers, *Aspergillus oryzae* cDNA prepared in (1) above as a template, and Premix Taq (manufactured by Takara Shuzo Co., Ltd.), PCR was carried out with a Program Temp Control System PC-700 (manufactured by Astec). After heating at 94°C for 5 minutes to denature template DNA, a cycle consisting of 2 minutes at 94°C, 30 seconds at 56°C, and 1.5 minutes at 72°C was repeated 30 times. The reaction solution was electrophoresed on 0.8% agarose gel. As a result, a DNA fragment with a size of approximately 850 bp was detected.

The DNA fragment was purified using a GENECLEAN Kit (manufactured by Q-Biogene) and then cleaved with restriction enzymes *Pst*I and *Eco*RI. The cleaved fragment was then ligated to a plasmid vector pRSET C for expression of prokaryotes (manufactured by Invitrogen), which had also been cleaved with *Pst*I and *Eco*RI. The ligation was carried out using a Ligation Kit Version 2 (manufactured by Takara Shuzo Co., Ltd.) *Escherichia coli* DH5α (manufactured by Takara Shuzo Co., Ltd.) was transformed with the obtained plasmid-containing solution by the calcium chloride method [J. Mol. Biol., 53, 159 (1970)], and transformants were selected in an LB agar plate medium (1% trypton, 0.5% yeast extract, 0.5% NaCl, 1.5% agar) containing 50 µg/ml ampicillin.

The growing cells (transformants) on this medium were subjected to a liquid culturing by common methods. Thereafter, plasmid DNA was extracted by common methods, and the plasmid was cleaved with *Pst*I and *Eco*RI. Thereafter, it was subjected to agarose gel electrophoresis, so that inserted fragments were confirmed. As a result, approximately 850 bp inserted fragments in addition to 2.9 kbp DNA fragments of the plasmid pRSET C were detected. This plasmid was named pPGP. The pPGP is a plasmid for expressing, under the control of a T7 promoter, a polypeptide comprising the amino acid sequence shown in SEQ ID NO: 10, in which 41 amino acid residues comprising a polyhistidine tag were added to the N terminus of pyroglutamyl peptidase of *Aspergillus oryzae.*

### Example 4 Production of pyroglutamyl peptidase by Escherichia coli

*Escherichia coli* BL21 was transformed with the plasmid pPGP for expression of pyroglutamyl peptidase, which had been constructed in Example 3(2), by the calcium method [J. Mol. Biol., 53, 159 (1970)], and transformants were selected in an LB agar plate medium (1% trypton, 0.5% yeast extract, 0.5% NaCl, 1.5% agar) containing 50 µg/ml ampicillin.

The obtained transformants were inoculated into 60 ml of an LB liquid medium (1% trypton, 0.5% yeast extract, 0.5% NaCl) and then allowed to grow at 25°C up to the logarithmic range. Thereafter, isopropyl-β-D-thiogalactopyranoside (hereinafter abbreviated as IPTG) was added thereto to a final concentration of 1 mmol/l. Thereafter, the mixture was further cultured at 25°C for 3 hours.

After completion of the culture, cell bodies were collected and suspended in 1 ml of a phosphate buffer solution (50 mmol/l Na₂HPO₄, 0.5 mol/l NaCl, pH 8). Thereafter, the cells were disrupted with ultrasonic wave, and were then centrifuged, so that insoluble fractions were eliminated and thus, a crude protein extract solution was obtained. The crude protein extract solution was mixed with 1 ml of an equilibrated Ni-NTA resin (manufactured by Invitrogen) with a concentration of 50%. The obtained mixture was incubated at 4°C for 30 minutes and then introduced into a column. The Ni-NTA resin was washed twice with 8 ml of a phosphate buffer solution containing 0.02 mol/l imidazole, and it was then eluted with 1 ml of a phosphate buffer solution containing 0.25 mol/l imidazole, so that approximately 35 kDa protein was purified as an almost single band on SDS-PAGE. Moreover, when the amount of the recovered protein was measured with a protein assay kit (manufactured by Biorad), it was found to be 91.8 µg.

Using pyroglutamic acid-paranitroanilide (hereinafter abbreviated as PCA-pNA) as a substrate, the activity of pyroglytamyl peptidase was measured. PCA-pNA was added to a 50 mmol/l phosphate buffer solution (pH 7.5) to a final concentration of 5 mmol/l, so as to prepare a substrate solution. 20 µl of an enzyme solution was added to 100 µl of the substrate solution. The mixture was reacted at 37°C for 10 minutes, and an absorbance at 405 nm was measured. The amount of free para-nitroaniline was calculated at a molar absorbance coefficient of 10500. 1 unit (U) was defined as an amount by which 1 µmol of para-nitroaniline is released at 37°C for 1 minute. The activity of a purified protein solution was measured by the above-described method. As a result, it was found that it had an activity of 6.6 mU/ml. On the other hand, when the activity of a solution obtained by performing the same above purification operation on cell bodies containing no IPTG was measured, it was found to be one fiftieth of the above obtained activity. Thus, it was found that pyroglutamyl peptidase activity was dependent on expression induction by IPTG. Accordingly, it was confirmed that a DNA fragment that has been inserted into pPGP encodes pyroglutamyl peptidase and it can thereby be used in production of pyroglutamyl peptidase, and that a polypeptide comprising the amino acid sequence shown in SEQ ID NO: 10 (an amino acid sequence in which 41 amino acid residues were added to the N terminus of the amino acid sequence shown in SEQ ID NO: 2) has a pyroglutamyl peptidase activity.

### Example 5 Production of protein hydrolysate by action of pyroglutamyl peptidase

1.0 g of Flavorzyme (manufactured by Novo Nordisk) was added to 200 ml of an aqueous solution containing 10% wheat gluten (Promic GT manufactured by Kyowa Hakko Kogyo Co., Ltd.), and the mixture was subjected to enzymolysis at 48°C for 3 days. Thereafter, the resultant product was filtrated and then treated by heating at 90°C, so as to obtain a protein hydrolysate.

*Escherichia coli* BL21 into which pPGP had been introduced was cultured in the same manner as in Example 4, so as to prepare a purified protein solution. The obtained purified protein solution having 0.5 U pyroglutamyl peptidase activity and a solution containing 0.1 g of Flavorzyme were added to 20 ml of a protein hydrolysate that had been filtrated using a membrane filter with a pore size of 0.2 µm, and the mixture was subjected to enzymolysis at 40°C for 2 days (Test A). The amount of total nitrogen, the amount of free amino acid, and the degradation rate of the above hydrolysed product were analyzed, and the obtained data were compared with those of a hydrolyzed product to which a solution having no pyroglutamyl peptidase activity and Flavorzyme had been added (Test B). The results are shown in Table 1.

**Table 1**

| | | A | B |
|---|---|---|---|
| Total nitrogen | (g/dl) | 1.31 | 1.27 |
| Free amino acid | (g/dl) | 0.56 | 0.49 |
| Degradation rate | (%) | 68.9 | 62.0 |

As is clear from the above results, a protein lysate with a high hydrolysis rate can be obtained by addition of the pyroglutamyl peptidase of the present invention.

### INDUSTRIAL APPLICABILITY

The present invention provides DNA encoding novel pyroglutamyl peptidase derived from *Aspergillus oryzae,* and pyroglutamyl peptidase which is produced by using the DNA. Using the pyroglutamyl peptidase, a protein lysate with a good flavor can be obtained at a high hydrolysis rate.

[Sequence Listing Free Text]
SEQ ID NO: 6 - an adaptor
SEQ ID NO: 7 - an adaptor
SEQ ID NO: 8 - a primer for amplification of pyroglutamyl peptidase cDNA of *Aspergillus oryzae*
SEQ ID NO: 9 - a primer for amplification of pyroglutamyl peptidase cDNA of *Aspergillus oryzae*
SEQ ID NO: 10 - an amino acid sequence, which comprises an addition of 41 amino acids comprising a polyhistidine tag to the N terminus of the amino acid sequence shown in SEQ ID NO: 2

SEQUENCE LISTING

<110> National Institute of Advanced Industrial Science and Technology
National Institute of Technology and Evaluation
National Research Institute of Brewing
<120> Pyroglutamyl peptidase and a gene thereof
<130> A21773A
<150> JP 2001-403261
<151> 2001-12-27
<160> 10
<170> PatentIn version 3.1

<210> 1
<211> 2201
<212> DNA
<213> Aspergillus oryzae
<220>
<223> Inventor: Machida, Masayuki; Abe, Keietsu; Gomi, Katsuya;
Inventor: Asai, Kiyoshi; Sano, Motoaki; Kin, Taishin
Inventor: Nagasaki, Hideki; Hosoyama, Akira; Akita, Osamu
Inventor: Ogasawara, Naotake; Kuhara, Satoru; Tokunaga, Chikara
Inventor: Toda, Itaru; Saitoh, Chiaki; Senoh, Akihiro
<220>
<221> source
<222> (1)..(2201)
<223> /organism="Aspergillus oryzae"
/strain="RIB 40"

<220>

<221> CDS

<222> (1001)..(1111)

<220>

<221> CDS

<222> (1197)..(1901)

<220>

<221> Intron

<222> (1112)..(1196)

<223> /number=1

<400> 1

tcaagatctg aatgataaca gatttcctag tccattgtaa cacatcgatg gggttgacgt 60

ggtaatagaa tcccaagact gatggaaatc gttctaggtg aagtcaaagt ctagatgatg 120

aataaacaac cagactctag gaaaatgctg gtctagaccc ttgggcgaga aggaatgtgc 180

tgataacgtc tcgctgcctt tcagcggtaa cgctaatcta aaagatcaac aaacaatcca 240

ggagcaacca gagcaatcgg tgcgtgttca gtaagtgagc agtgagtgca caggagcact 300

cacgtgctaa ccttacaaaa gcagcggcac ccatatcaaa caggaagaag tgggccgtac 360

ggtagttcta ggatgacata ccgaaacccc ttatttgttc gcttaaatag atccctgccc 420

agctttactg atggatttct aatcgcaaag taattgggtg aaataccatc ggtattaacc 480

tagtgaatgg tgattctcaa ccatcgagta caagtcattc tcactattga actttccaaa 540

aagccccgtg aacaagcagt ctgcggtttg ccccggctga agcaaggggg aaattgtcgg 600

tcaggactcg gaacttcgga agcgaagcag aatcggcggt ggccaaaagg catgcgacgt 660

gacagcacct cacatcattc cgggacaata acataggttc aattgcacaa ttgtctcaag 720

aacatgggtg attgtcagat tgatacgtca atcaagcttt gtgggcggtc aagatgaggg 780

gaggtcatgt gccttatcac cttatcgata tcgatatcgc gtgatgccaa gacctgcatg 840

cggtggttgt aatgcggggg aagctccgtc gatatcttga atatatcttt agtccctcct 900

ctctatcctt tttgtggcgt acatagctac cgtgtatata cgaagtaaag gcgttgttcc 960

caccactgat tcctagcttg ccttgaccta tccactagcc atg gga gac ttt ggc 1015
Met Gly Asp Phe Gly
1               5
cca ccg gtg cca ata ccc gag acg gag gta att ggt ctt gct tcg tca 1063
Pro Pro Val Pro Ile Pro Glu Thr Glu Val Ile Gly Leu Ala Ser Ser
                10                  15                  20
tct ttg aca gat cca gaa gag gtc tcg gta ctg gtg aca ggg ttc ggt 1111
Ser Leu Thr Asp Pro Glu Glu Val Ser Val Leu Val Thr Gly Phe Gly
            25                  30                  35
gtaagtttat ctgtcttatg cttggcttat gtttctttga ccgtcttggc ttctgattcg 1171
gccctcaaat gctaaaatat actag cca ttc aag acc aac cta gtc aat gcc 1223
Pro Phe Lys Thr Asn Leu Val Asn Ala
        40                  45
tcg tat ttg att gcc tca tct ctg cca gag tcg ctt gac ctt cct tcg 1271
Ser Tyr Leu Ile Ala Ser Ser Leu Pro Glu Ser Leu Asp Leu Pro Ser
            50                  55                  60
gcg aag ccg tct gga tcc ggg cct act tct cgt cgg att tca att cat 1319
Ala Lys Pro Ser Gly Ser Gly Pro Thr Ser Arg Arg Ile Ser Ile His
        65                  70                  75
gtc cat cca tcg ccc att ccc gtc gct tac tca aca gtg cgg aca act 1367
Val His Pro Ser Pro Ile Pro Val Ala Tyr Ser Thr Val Arg Thr Thr
    80                  85                  90
att cca acc atc cta gag gat tac gcc aag tcc cat gga ggt cga cga 1415
Ile Pro Thr Ile Leu Glu Asp Tyr Ala Lys Ser His Gly Gly Arg Arg
95                  100                 105                 110
cca gac att gta ctc cat atg gga ata gcg gct aca aga tcg tac tac 1463
Pro Asp Ile Val Leu His Met Gly Ile Ala Ala Thr Arg Ser Tyr Tyr

                115                 120                 125

tcg att gag acc aag gcg cat cga gat tct tac cac ttg tcc gat atc 1511

Ser Ile Glu Thr Lys Ala His Arg Asp Ser Tyr His Leu Ser Asp Ile

            130                 135                 140

aaa ggc aga atc ggt tat gaa gat ggg gag aag gtt tgg agg gag cag 1559

Lys Gly Arg Ile Gly Tyr Glu Asp Gly Glu Lys Val Trp Arg Glu Gln

        145                 150                 155

cag ctc ccg cca gta ctc cag gct ggt cct gcg gcg gat tcc aca gac 1607

Gln Leu Pro Pro Val Leu Gln Ala Gly Pro Ala Ala Asp Ser Thr Asp

    160                 165                 170

gta gta cgg aaa gtt ctc cac ccc cag ccg ccc aat gac gac ttt ctc 1655

Val Val Arg Lys Val Leu His Pro Gln Pro Pro Asn Asp Asp Phe Leu

175                 180                 185                 190

aac acg tgg aag tcg ttt gta tct cct gga gca gac gtc cgg ata tcc 1703

Asn Thr Trp Lys Ser Phe Val Ser Pro Gly Ala Asp Val Arg Ile Ser

                195                 200                 205

gag gac gct gga cgc tac ctc tgc gag ttc atc ttt tat aca agt ctg 1751

Glu Asp Ala Gly Arg Tyr Leu Cys Glu Phe Ile Phe Tyr Thr Ser Leu

            210                 215                 220

gcc cag gcg ttt caa caa ggc cag cac cga aac gtc gtt ttc ttc cat 1799

Ala Gln Ala Phe Gln Gln Gly Gln His Arg Asn Val Val Phe Phe His

        225                 230                 235

gtg cct gga tct tgc gcc gac gag gac atc gag aga ggc acg gat att 1847

Val Pro Gly Ser Cys Ala Asp Glu Asp Ile Glu Arg Gly Thr Asp Ile

    240                 245                 250

gca gct gga ttg atc aaa gct ctt gta aga tgt tgg gtt agc gag cag 1895

Ala Ala Gly Leu Ile Lys Ala Leu Val Arg Cys Trp Val Ser Glu Gln

255                 260                 265                 270

gta tag agcggcatgc aggttgctgg tatcgttttg caaagcaaga gcatgggcac 1951

Val

tggacgatat atatacttgc atttctatgg cgcggtgcac tatctgggtt ccggatgcgc 2011

ttttagctgc agtcactcgt gatcatttat ttatagggga cttctgtccc cggtcttttc 2071

aggttgagtt atacatgttt cacaggtttt ggatacacta tttaccctct gactactatc 2131

gatgaatata gacagttgtc aagcatgata ttgggttcta ccgtattcgt atatgtgtag 2191

aatttcccgt 2201

<210> 2

<211> 271

<212> PRT

<213> Aspergillus oryzae

<400> 2

Met Gly Asp Phe Gly Pro Pro Val Pro Ile Pro Glu Thr Glu Val Ile

1               5                   10                  15

Gly Leu Ala Ser Ser Ser Leu Thr Asp Pro Glu Glu Val Ser Val Leu

            20                  25                  30

Val Thr Gly Phe Gly Pro Phe Lys Thr Asn Leu Val Asn Ala Ser Tyr

        35                  40                  45

Leu Ile Ala Ser Ser Leu Pro Glu Ser Leu Asp Leu Pro Ser Ala Lys

    50                  55                  60

Pro Ser Gly Ser Gly Pro Thr Ser Arg Arg Ile Ser Ile His Val His

65                  70                  75                  80

Pro Ser Pro Ile Pro Val Ala Tyr Ser Thr Val Arg Thr Thr Ile Pro

                85                  90                  95

Thr Ile Leu Glu Asp Tyr Ala Lys Ser His Gly Gly Arg Arg Pro Asp

            100                 105                 110
Ile Val Leu His Met Gly Ile Ala Ala Thr Arg Ser Tyr Tyr Ser Ile
        115                 120                 125
Glu Thr Lys Ala His Arg Asp Ser Tyr His Leu Ser Asp Ile Lys Gly
    130                 135                 140
Arg Ile Gly Tyr Glu Asp Gly Glu Lys Val Trp Arg Glu Gln Gln Leu
145                 150                 155                 160
Pro Pro Val Leu Gln Ala Gly Pro Ala Ala Asp Ser Thr Asp Val Val
                165                 170                 175
Arg Lys Val Leu His Pro Gln Pro Pro Asn Asp Asp Phe Leu Asn Thr
            180                 185                 190
Trp Lys Ser Phe Val Ser Pro Gly Ala Asp Val Arg Ile Ser Glu Asp
        195                 200                 205
Ala Gly Arg Tyr Leu Cys Glu Phe Ile Phe Tyr Thr Ser Leu Ala Gln
    210                 215                 220
Ala Phe Gln Gln Gly Gln His Arg Asn Val Val Phe Phe His Val Pro
225                 230                 235                 240
Gly Ser Cys Ala Asp Glu Asp Ile Glu Arg Gly Thr Asp Ile Ala Ala
                245                 250                 255
Gly Leu Ile Lys Ala Leu Val Arg Cys Trp Val Ser Glu Gln Val
            260                 265                 270

<210> 3
<211> 1000
<212> DNA
<213> Aspergillus oryzae
<220>

<221> source

<222> (1)..(1000)

<223> /organism="Aspergillus oryzae"

/strain="RIB 40"

<400> 3

tcaagatctg aatgataaca gatttcctag tccattgtaa cacatcgatg gggttgacgt 60

ggtaatagaa tcccaagact gatggaaatc gttctaggtg aagtcaaagt ctagatgatg 120

aataaacaac cagactctag gaaaatgctg gtctagaccc ttgggcgaga aggaatgtgc 180

tgataacgtc tcgctgcctt tcagcggtaa cgctaatcta aaagatcaac aaacaatcca 240

ggagcaacca gagcaatcgg tgcgtgttca gtaagtgagc agtgagtgca caggagcact 300

cacgtgctaa ccttacaaaa gcagcggcac ccatatcaaa caggaagaag tgggccgtac 360

ggtagttcta ggatgacata ccgaaacccc ttatttgttc gcttaaatag atccctgccc 420

agctttactg atggatttct aatcgcaaag taattgggtg aaataccatc ggtattaacc 480

tagtgaatgg tgattctcaa ccatcgagta caagtcattc tcactattga actttccaaa 540

aagccccgtg aacaagcagt ctgcggtttg ccccggctga agcaaggggg aaattgtcgg 600

tcaggactcg gaacttcgga agcgaagcag aatcggcggt ggccaaaagg catgcgacgt 660

gacagcacct cacatcattc cgggacaata acataggttc aattgcacaa ttgtctcaag 720

aacatgggtg attgtcagat tgatacgtca atcaagcttt gtgggcggtc aagatgaggg 780

gaggtcatgt gccttatcac cttatcgata tcgatatcgc gtgatgccaa gacctgcatg 840

cggtggttgt aatgcggggg aagctccgtc gatatcttga atatatcttt agtccctcct 900

ctctatcctt tttgtggcgt acatagctac cgtgtatata cgaagtaaag gcgttgttcc 960

caccactgat tcctagcttg ccttgaccta tccactagcc 1000

<210> 4

<211> 300

<212> DNA

<213> Aspergillus oryzae

<220>

<221>  source

<222>  (1)..(300)

<223>  /organism="Aspergillus oryzae"

       /strain="RIB 40"

<400>  4

agcggcatgc aggttgctgg tatcgttttg caaagcaaga gcatgggcac tggacgatat    60

atatacttgc atttctatgg cgcggtgcac tatctgggtt ccggatgcgc ttttagctgc   120

agtcactcgt gatcatttat ttatagggga cttctgtccc cggtcttttc aggttgagtt   180

atacatgttt cacaggtttt ggatacacta tttaccctct gactactatc gatgaatata   240

gacagttgtc aagcatgata ttgggttcta ccgtattcgt atatgtgtag aatttcccgt   300


<210>  5

<211>  816

<212>  DNA

<213>  Aspergillus oryzae

<220>

<221>  source

<222>  (1)..(816)

<223>  /organism="Aspergillus oryzae"

       /strain="RIB 40"

<220>

<221>  CDS

<222>  (1)..(816)

<400>  5

atg gga gac ttt ggc cca ccg gtg cca ata ccc gag acg gag gta att      48

Met Gly Asp Phe Gly Pro Pro Val Pro Ile Pro Glu Thr Glu Val Ile

1                   5                   10                  15
ggt ctt gct tcg tca tct ttg aca gat cca gaa gag gtc tcg gta ctg     96
Gly Leu Ala Ser Ser Ser Leu Thr Asp Pro Glu Glu Val Ser Val Leu
                20                  25                  30
gtg aca ggg ttc ggt cca ttc aag acc aac cta gtc aat gcc tcg tat     144
Val Thr Gly Phe Gly Pro Phe Lys Thr Asn Leu Val Asn Ala Ser Tyr
            35                  40                  45
ttg att gcc tca tct ctg cca gag tcg ctt gac ctt cct tcg gcg aag     192
Leu Ile Ala Ser Ser Leu Pro Glu Ser Leu Asp Leu Pro Ser Ala Lys
    50                  55                  60
ccg tct gga tcc ggg cct act tct cgt cgg att tca att cat gtc cat     240
Pro Ser Gly Ser Gly Pro Thr Ser Arg Arg Ile Ser Ile His Val His
65                  70                  75                  80
cca tcg ccc att ccc gtc gct tac tca aca gtg cgg aca act att cca     288
Pro Ser Pro Ile Pro Val Ala Tyr Ser Thr Val Arg Thr Thr Ile Pro
                85                  90                  95
acc atc cta gag gat tac gcc aag tcc cat gga ggt cga cga cca gac     336
Thr Ile Leu Glu Asp Tyr Ala Lys Ser His Gly Gly Arg Arg Pro Asp
                100                 105                 110
att gta ctc cat atg gga ata gcg gct aca aga tcg tac tac tcg att     384
Ile Val Leu His Met Gly Ile Ala Ala Thr Arg Ser Tyr Tyr Ser Ile
            115                 120                 125
gag acc aag gcg cat cga gat tct tac cac ttg tcc gat atc aaa ggc     432
Glu Thr Lys Ala His Arg Asp Ser Tyr His Leu Ser Asp Ile Lys Gly
    130                 135                 140
aga atc ggt tat gaa gat ggg gag aag gtt tgg agg gag cag cag ctc     480
Arg Ile Gly Tyr Glu Asp Gly Glu Lys Val Trp Arg Glu Gln Gln Leu

145                 150                 155                 160

ccg cca gta ctc cag gct ggt cct gcg gcg gat tcc aca gac gta gta     528

Pro Pro Val Leu Gln Ala Gly Pro Ala Ala Asp Ser Thr Asp Val Val

                165                 170                 175

cgg aaa gtt ctc cac ccc cag ccg ccc aat gac gac ttt ctc aac acg     576

Arg Lys Val Leu His Pro Gln Pro Pro Asn Asp Asp Phe Leu Asn Thr

            180                 185                 190

tgg aag tcg ttt gta tct cct gga gca gac gtc cgg ata tcc gag gac     624

Trp Lys Ser Phe Val Ser Pro Gly Ala Asp Val Arg Ile Ser Glu Asp

        195                 200                 205

gct gga cgc tac ctc tgc gag ttc atc ttt tat aca agt ctg gcc cag     672

Ala Gly Arg Tyr Leu Cys Glu Phe Ile Phe Tyr Thr Ser Leu Ala Gln

    210                 215                 220

gcg ttt caa caa ggc cag cac cga aac gtc gtt ttc ttc cat gtg cct     720

Ala Phe Gln Gln Gly Gln His Arg Asn Val Val Phe Phe His Val Pro

225                 230                 235                 240

gga tct tgc gcc gac gag gac atc gag aga ggc acg gat att gca gct     768

Gly Ser Cys Ala Asp Glu Asp Ile Glu Arg Gly Thr Asp Ile Ala Ala

                245                 250                 255

gga ttg atc aaa gct ctt gta aga tgt tgg gtt agc gag cag gta tag     816

Gly Leu Ile Lys Ala Leu Val Arg Cys Trp Val Ser Glu Gln Val

            260                 265                 270

<210>  6

<211>  16

<212>  DNA

<213>  Artificial

<220>

<223>  adaptor

<400>  6

cgagagcggc cgctac                                                    16

<210>  7

<211>  13

<212>  DNA

<213>  Artificial

<220>

<223>  adaptor

<400>  7

gtagcggccg ctc                                                       13

<210>  8

<211>  29

<212>  DNA

<213>  Artificial

<220>

<223>  primer for amplification of Aspergillus oryzae pyroglutamyl pepti

       dase cDNA

<400>  8

gggctgcaga tgggagactt tggcccacc                                      29

<210>  9

<211>  30

<212>  DNA

<213>  Artificial

<220>

<223>  primer for amplification of Aspergillus oryzae pyroglutamyl pepti
       dase cDNA

<400>  9
ggggaattcc tatacctgct cgctaaccca                                        30


<210>  10

<211>  312

<212>  PRT

<213>  Artificial

<220>

<223>  amino acid sequence wherein 41 amino acids containing polyhistidi
       ne-tag are added at N-terminal of amino acid sequence of SEQ ID N
       O:2

<400>  10

Met Arg Gly Ser His His His His His His Gly Met Ala Ser Met Thr
1               5                   10                  15

Gly Gly Gln Gln Met Gly Arg Asp Leu Tyr Asp Asp Asp Asp Lys Asp
            20                  25                  30

Arg Trp Ile Arg Pro Arg Asp Leu Gln Met Gly Asp Phe Gly Pro Pro
        35                  40                  45

Val Pro Ile Pro Glu Thr Glu Val Ile Gly Leu Ala Ser Ser Ser Leu
    50                  55                  60

Thr Asp Pro Glu Glu Val Ser Val Leu Val Thr Gly Phe Gly Pro Phe
65                  70                  75                  80

Lys Thr Asn Leu Val Asn Ala Ser Tyr Leu Ile Ala Ser Ser Leu Pro

                85                  90                  95
Glu Ser Leu Asp Leu Pro Ser Ala Lys Pro Ser Gly Ser Gly Pro Thr
            100                 105                 110
Ser Arg Arg Ile Ser Ile His Val His Pro Ser Pro Ile Pro Val Ala
        115                 120                 125
Tyr Ser Thr Val Arg Thr Thr Ile Pro Thr Ile Leu Glu Asp Tyr Ala
    130                 135                 140
Lys Ser His Gly Gly Arg Arg Pro Asp Ile Val Leu His Met Gly Ile
145                 150                 155                 160
Ala Ala Thr Arg Ser Tyr Tyr Ser Ile Glu Thr Lys Ala His Arg Asp
                165                 170                 175
Ser Tyr His Leu Ser Asp Ile Lys Gly Arg Ile Gly Tyr Glu Asp Gly
            180                 185                 190
Glu Lys Val Trp Arg Glu Gln Gln Leu Pro Pro Val Leu Gln Ala Gly
        195                 200                 205
Pro Ala Ala Asp Ser Thr Asp Val Val Arg Lys Val Leu His Pro Gln
    210                 215                 220
Pro Pro Asn Asp Asp Phe Leu Asn Thr Trp Lys Ser Phe Val Ser Pro
225                 230                 235                 240
Gly Ala Asp Val Arg Ile Ser Glu Asp Ala Gly Arg Tyr Leu Cys Glu
                245                 250                 255
Phe Ile Phe Tyr Thr Ser Leu Ala Gln Ala Phe Gln Gln Gly Gln His
            260                 265                 270
Arg Asn Val Val Phe Phe His Val Pro Gly Ser Cys Ala Asp Glu Asp
        275                 280                 285
Ile Glu Arg Gly Thr Asp Ile Ala Ala Gly Leu Ile Lys Ala Leu Val
    290                 295                 300

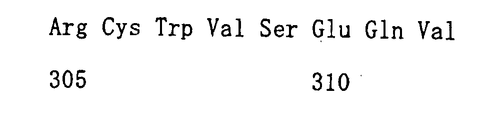

## Claims

1. A polypeptide selected from the group consisting of the following (a) to (c):
(a) a polypeptide comprising an amino acid sequence shown in SEQ ID NO: 2,
(b) a polypeptide comprising an amino acid sequence substantially identical to the amino acid sequence shown in SEQ ID NO: 2, and having a pyroglutamyl peptidase activity, and
(c) a polypeptide comprising an amino acid sequence wherein one or more amino acid residues are deleted, substituted or added in the amino acid sequence shown in SEQ ID NO: 2, and having a pyroglutamyl peptidase activity.

2. DNA comprising a nucleotide sequence encoding the polypeptide according to claim 1.

3. DNA selected from the group consisting of the following (a) to (c):
(a) DNA comprising a nucleotide sequence shown in SEQ ID NO: 1,
(b) DNA comprising a nucleotide sequence shown in SEQ ID NO: 5, and
(c) DNA which hybridizes with DNA comprising a nucleotide sequence complementary to the nucleotide sequence shown in SEQ ID NO: 1 or 5 under stringent conditions, and comprises a nucleotide sequence encoding a polypeptide having a pyroglutamyl peptidase activity.

4. DNA selected from the group consisting of the following (a) to (c):
(a) DNA comprising a nucleotide sequence shown in SEQ ID NO: 3,
(b) DNA which comprises a partial sequence of the nucleotide sequence shown in SEQ ID NO: 3 consisting of 100 or more nucleotides and functions as a promoter, and
(c) DNA which hybridizes with DNA comprising a nucleotide sequence complementary to the nucleotide sequence shown in SEQ ID NO: 3 under stringent conditions and functions as a promoter.

5. DNA selected from the group consisting of the following (a) to (c):
(a) DNA comprising a nucleotide sequence complementary to a nucleotide sequence shown in SEQ ID NO: 4,
(b) DNA which comprises a partial sequence of a nucleotide sequence complementary to the nucleotide sequence shown in SEQ ID NO: 4 consisting of 15 or more nucleotides, and
(c) DNA which hybridizes with DNA comprising the nucleotide sequence shown in SEQ ID NO: 4 under stringent conditions.

6. The DNA according to any one of claims 2 to 5, wherein DNA is genomic DNA.

7. An oligonucleotide comprising a nucleotide sequence consisting of 15 or more contiguous nucleotides of the nucleotide sequence of the DNA according to any one of claims 2 to 6 or a nucleotide sequence complementary thereto.

8. A recombinant DNA comprising the DNA according to claims 2 or 3.

9. A transformant comprising the recombinant DNA according to claim 8.

10. A process for producing the polypeptide according to claim 1, which comprises culturing a microorganism having an ability to produce the polypeptide in a medium, so as to produce and accumulate the polypeptide in a culture, and recovering the polypeptide from the culture.

11. The process according to claim 10, wherein the microorganism is the transformant according to claim 9.

12. The process according to claim 10, wherein the microorganism is filamentous fungus.

13. The process according to claim 12, wherein the filamentous fungus belongs to one genus selected from a group consisting of *Aspergillus, Penicillium, Humicola, Trichodenna, Mucor,* and *Fusarium.*

14. The process according to claim 13, wherein the filamentous fungus belonging to *Aspergillus* belongs to one species selected from a group consisting of *Aspergillus oryzae, Aspergillus sojae, Aspergillus niger, Aspergillus awamori, Aspergillus kawachii,* *Aspergillus parasiticus, Aspergillus flavus*, *Aspergillus nomius, Aspergillus fumigatus,* and *Aspergillus nidulans.*

15. A process for producing a protein hydrolysate, which comprises adding the polypeptide according to claim 1 and a protein hydrolase to a raw material containing a protein, and degrading the protein.

16. A process for producing a protein hydrolysate, which comprises adding a culture containing the polypeptide according to claim 1 which is obtained by culturing a microorganism having an ability to produce the polypeptide according to claim 1 in a medium, or a treated product thereof, and a protein hydrolase, to a raw material containing a protein, and degrading the protein.

17. The process according to claim 16, wherein the microorganism is the transformant according to claim 9.

18. The process according to claim 16, wherein the microorganism is filamentous fungus.

19. The process according to claim 18, wherein the filamentous fungus belongs to one genus selected from a group consisting of *Aspergillus, Penicillium, Humicola, Trichoderma, Mucor,* and *Fusarium.*

20. The process according to claim 19, wherein the filamentous fungus belonging to *Aspergillus* belongs to one species selected from a group consisting of *Aspergillus oryzae, Aspergillus sojae, Aspergillus niger, Aspergillus awamori, Aspergillus kawachii, Aspergillus parasiticus, Aspergillus flavus, Aspergillus nomius, Aspergillus fumigatus,* and *Aspergillus nidulans.*

21. A protein hydrolysate which is produced by the process according to any one of claims 15 to 20.

22. An antibody which specifically binds to the polypeptide according to claim 1.

23. A method of detecting or quantifying the polypeptide according to claim 1 which comprises using the antibody according to claim 22.
